# EUROPEAN PATENT APPLICATION

(11) **EP 1 932 870 A1**
(43) Date of publication of application: **18.06.2008**
(21) Application number: 06811467.7
(22) Date of filing: 02.10.2006
(51) Int. Cl.: C08G 81/00, A61K 9/08, A61K 9/10, A61K 9/127, A61K 9/50, A61K 47/34

(54) **BIOCOMPATIBLE BLOCK COPOLYMER, USE THEREOF, AND PRODUCTION METHOD THEREOF**

(30) Priority: 05.10.2005 JP 2005292451
(71) Applicant: Tokyo Cro, Inc., Bunkyo-ku, Tokyo 112-0004 (JP); National University Corporation Gunma University, Gunma 371-0044 (JP)
(72) Inventor: KATAKAI, Ryoichi, Gunma 376-0001 (JP); KOHAMA, Kazuhiro, Gunma 371-0031 (JP); SUZUKI, Yoshiki, Tokyo 191-0052 (JP)
(74) Representative: Albrecht, Thomas
(86) International application number: PCT/JP2006/320140
(87) International publication number: WO 2007/043486

(57) **Abstract**

A biocompatible block copolymer having a hydrophobic segment composed of amino acid and hydroxycarboxylic acid and a hydrophilic segment composed of polyalkylene oxide, wherein the terminal not bonded to the hydrophilic segment of the hydrophobic segment is composed of an amino acid unit.

## Description

### TECHNICAL FIELD

The present invention relates to a biocompatible block copolymer having a hydrophobic segment composed of amino acid and hydroxycarboxylic acid and a hydrophilic segment composed of polyalkylene oxide, a micelle, particulate or composite which is composed of the biocompatible block copolymer and a drug, use thereof, a pharmaceutical composition comprising the same, and a method of manufacturing the same. This micelle, particulate or composite enables the solubilization, dispersion, stabilization, sustained-release and delivery to a focal site of a drug.

### BACKGROUND ART

A drug produces its effect when it reaches its active site in vivo. There are various factors that the drug cannot reach its active site. For example, (1) the drug is hardly soluble in a body fluid, (2) it is unstable under physiological conditions such as an enzyme, pH and others, (3) it cannot pass through a membranous barrier such as endothelium or mucosa, (4) it causes an undesired immune reaction, (5) it disappears or is excreted from the blood swiftly, and (6) it lacks targeting ability for a target site.

As results caused by these, the following problems occur. For example, (1) it is difficult to prepare an injection solution, (2) the oral administration of a proteinaceous medicine is difficult, (3) the delivery of a drug to a cancer tissue, brain or nervous tissue is unsatisfactory, (4) the mucosal absorption of a hydrophilic drug is unsatisfactory, (5) the bioavailability of a hydrophobic drug is unsatisfactory, (6) cytokine disappears in the blood too quickly, and (7) there is a lack of transfer ability to a target site.

To solve the above problems, a large number of approaches are proposed. For example, (1) the formation of a bonded form of a hydrophilic polymer compound and a proteinaceous drug, (2) the formation of a bonded form of a passive transport substance and a drug, (3) the encapsulation of a hydrophilic drug in a liposome, (4) the encapsulation of a drug in a nanometric or micrometric biodegradable polymer particulate, and (5) the formation of a polymer micelle from an amphiphilic polymer substance and a drug. However, a universal solution to all the problems related to the delivery of a drug is not found yet.

An example of the biodegradable polymer is polyamino acid in which the main chains are interconnected by an amide bond. However, the amide bond strongly forms hydrogen bond between molecules or in the molecule to form a stable high-order structure. Therefore, to decompose a polyamide by biodegradation, the high-order structure supported by this strong hydrogen bond must be broken, and it has been revealed that the polyamide cannot be completely decomposed except a racemic material having an unstable structure and a special polyamino acid having an ionized polar side chain (refer to M. Asano, M. Yoshida, I. Kaetsu, K. Nakai, H. Yamanaka, H. Yuasa, K. Shiba, K. Suzuki, M. Oya, Mikromol. Chem., (1983), 84:1761).

As an example of a polymer for delivering a biodegradable drug is a polyhydroxy acid obtained by the ester condensation of a hydroxycarboxylic acid having a hydroxyl group and a carboxyl group in the molecule. Since the ester bond has no hydrogen atom in the polyhydroxy acid, it has been made clear that a hydrogen bond is not produced in the ester bond and that polyhydroxy acid is biodegraded more easily than polyamino acid. Further, a method of polymerizing a hydroxycarboxylic acid by using a hydroxycarboxylic acid and water without a catalyst has been developed. When a polymer obtained by polycondensing an oligomer without the inclusion of the residue of the catalyst is used in a drug delivery system, there is no possibility of inducing a foreign body reaction as compared with a polymer which is formed by using a metal catalyst, and the polymer can be molded easily due to weak interaction between molecules. Therefore, the polymer is a preferred material in the drug delivery system. Polyhydroxy acid has a higher biodegradation rate as its molecular weight becomes lower. Particularly, low molecular weight poly-DL-lactic acid is used as a preferred drug delivery system (M. Asano, H. Fukuzaki, M. Yoshida, M. Kumakura, T. Mashimo, H. Yuasa, K. Imai, H. Yamanaka, K. Suzuki, J. Controlled Release, (1989) 9: 111, M. Asano, H. Fukuzaki, M. Yoshida, M. Kumakura, T. Mashimo, H. Yuasa, K. Imai, H. Yamanaka, Biomaterials, (1989) 10; 569, and T. Mashimo, H. Yuasa, K. Imai, H. Yamanaka, M. Asano, M. Yoshida, M. Kumakura, Kita Kanto Igaku (North Kanto Medical Science) 41, (1991) 311).

Meanwhile, a polymer micelle is basically a nano-particle which has a hydrophilic segment as the outer shell and a hydrophobic segment as the inner core, and a large number of polymer micelles have been reported as carriers for the solubilization, stabilization, sustained-release and delivery of a drug. For example, a drug carrier which is a block copolymer consisting of a hydrophilic segment such as polyalkylene oxide and a hydrophobic segment such as polyalkyl aspartate (refer to JP-A 06-107565 JP-A 06-206830, JP-A 06-206832, JP-A 11-100331, JP-A 2001-226294, JP-A 2003-342167, JP-A 2004-010479, JP-A 2004-352972, JP-A 2005-029480, JP-B 2005-501831 and WO03/000771), a particulate of a block copolymer consisting of a hydrophilic segment such as hydrophilic polyamino acid and a hydrophobic segment such as polylactide (refer to JP-A 11-269097), a block copolymer comprising a hydrophilic segment such as polyethylene glycol and a charged segment such as polyamine or polycarboxylic acid (refer to JP-A 08-188541, JP-A 2001-146556, JP-A 2005-008614 and JP-B 2001-525357), a method of preparing a polymer micelle containing a drug hardly soluble in water (refer to JP-A 11-335267, JP-A 2003-012505, JP-A 2003-026566, JP-A 2003-026812, JP-A 2003-342168, JP-B 2003-532688 and WO02/026241) have been reported.

However, there is no description of a biocompatible block copolymer having a hydrophobic segment composed of amino acid and hydroxycarboxylic acid and a hydrophilic segment composed of polyalkylene oxide and the formation of a micelle, particulate and composite comprising the copolymer in all of the above documents.

Further, a depsipeptide copolymer obtained by polymerizing optically active 3-substitution-2,5-morpholinedione and cyclic lactone, a biologically absorbing surgical device manufactured from polydepsipeptide (refer to JP-A 7-188411) and a biodegradable polylactone ester amide having a specific polyamide unit, a specific polyester unit and a specific polylactone unit (refer to JP-A 11-35679) have been reported as biodegradable plastics comprising amino acid and hydroxycarboxylic acid. It is known that a polymer comprising specific depsipeptide and polylactic acid has excellent biodegradability and can improve flexibility while retaining strength (refer to JP-A 2001-31762) and that a biodegradable copolymer obtained by the ring-opening polymerization of lactide and ε-caprolactone having at least one reactive substituent selected from the group consisting of a hydroxyl group, amino group and carboxyl group has high moldability and functionality based on its use (refer to JP-A 2002-234934) . One of the inventors of the present invention discloses a method of synthesizing depsipeptide and a synthetic product (refer to JP-A 2004-269462).

However, there is no description of a biocompatible block copolymer having a hydrophobic segment composed of amino acid and hydroxycarboxylic acid and a hydrophilic segment composed of polyalkylene oxide and of the formation of a micelle, particulate and composite comprising the copolymer in all the documents of JP-A 7-188411, JP-A 11-35679, JP-A 2001-31762, JP-A 2002-234934 and JP-A 2004-269462.

Further, a 3-dimensional block copolymer is reported as a biodegradable plastic consisting of a block composed of amino acid and hydroxycarboxylic acid and a block composed of polyalkylene glycol (refer to the pamphlet of WO2005/003214) .

However, there is no description of a biocompatible block copolymer having a hydrophobic segment composed of amino acid and hydroxycarboxylic acid and a hydrophilic segment composed of polyalkylene oxide and the formation of a micelle, particulate and composite comprising the copolymer in the pamphlet of WO2005/003214 as well.

### DISCLOSURE OF THE INVENTION

It is an object of the present invention to provide a biocompatible block copolymer which can eliminate the above six factors that a drug cannot reach its active site and has affinity for a drug.

It is another object of the present invention to provide a biocompatible block copolymer having an ester bond which is easily broken in vivo and an amide bond which is hardly broken, showing no antigenicity, whose affinity for a drug can be adjusted by controlling the types, numbers and sequences of amino acids and hydroxycarboxylic acids.

It is still another object of the present invention to provide a micelle, particulate or composite which comprises the above biocompatible block copolymer and a drug and enables the solubilization, dispersion, stabilization, sustained-release and delivery of the drug to a required site in vivo.

It is a further object of the present invention to provide a method of manufacturing the above micelle, particulate or composite advantageously.

Other objects and advantages of the present invention will become apparent from the following description.

According to the present invention, firstly, the above objects and advantages of the present invention are attained by a biocompatible block copolymer having a hydrophobic segment composed of amino acid and hydroxycarboxylic acid and a hydrophilic segment composed of polyalkylene oxide, wherein the terminal not bonded to the hydrophilic segment of the hydrophobic segment is composed of an amino acid unit.

According to the present invention, secondly, the above objects and advantages of the present invention are attained by a micelle comprising the biocompatible block copolymer of the present invention and a drug.

According to the present invention, thirdly, the above objects and advantages of the present invention are attained by a particulate or composite (excluding a micelle) comprising the biocompatible block copolymer of the present invention and a drug.

According to the present invention, in the fourth place, the above objects and advantages of the present invention are attained by a pharmaceutical composition comprising the micelle, particulate or composite of the present invention.

According to the present invention, in the fifth place, the above objects and advantages of the present invention are attained by a method of manufacturing a micelle or composite comprising a drug, comprising the step of mixing together a biocompatible block copolymer having a hydrophobic segment composed of amino acid and hydroxycarboxylic acid and a hydrophilic segment composed of polyalkylene oxide and a drug in water.

According to the present invention, finally, the above objects and advantages of the present invention are attained by a method of manufacturing a micelle, particulate or composite, comprising the steps of preparing a mixture of a biocompatible block copolymer having a hydrophobic segment composed of amino acid and hydroxycarboxylic acid and a hydrophilic segment composed of polyalkylene oxide, a drug and a solvent, and removing the solvent from the mixture.

### BEST MODE FOR CARRYING OUT THE INVENTION

The biocompatible block copolymer of the present invention has a hydrophobic segment and a hydrophilic segment. In this text, the term "biocompatible" denotes the property of becoming compatible with a living tissue and not damaging a living body after it is administered to the inside of the living body. For instance, even when it is destructively metabolized or not decomposed in vivo, it is excreted to the outside of the body in the end. In this text, the term "biodegradable" denotes the property of being destructively metabolized in a living tissue and not damaging a living body after it is administered to the inside of the living body. For instance, it is destructively metabolized in vivo and excreted to the outside of the body in the end. In this text, the term "hydrophobic segment" denotes a biodegradable polymer or a derivative thereof which is hardly soluble or insoluble in water and is a high molecular weight polymer more hydrophobic than the hydrophilic segment as the other component forming the block copolymer. In this text, the term "hydrophilic segment" denotes a high molecular weight polymer or a derivative thereof which is hydrophilic for the hydrophobic segment of the block copolymer even when it is soluble or hardly soluble in water. In this text, the term "micelle" denotes a particle having an inner core portion and an outer shell portion which differ from each other in composition. In this text, the term "particulate" denotes particles which have an average particle diameter of 300 µm or less, do not have clear-cut inner core and outer shell portions and contain a drug almost uniformly dispersed in the block copolymer. In this text, the term "composite" denotes a composite which does not have clear-cut inner core and outer shell portions and is formed by interaction between drug molecules or drug crystals and the block copolymer. In this text, the term "ligand" denotes all molecules which specifically bind to a specific target molecule to form a bound composite and a leading portion headed to a target.

The biocompatible block copolymer of the present invention consists of a biodegradable block composed of amino acid and hydroxycarboxylic acid as a hydrophobic segment and a biocompatible block composed of polyalkylene oxide as a hydrophilic segment. Stated more specifically, it is a block copolymer having the hydrophilic segment covalently bonded to the terminal of the biodegradable hydrophobic segment. These segments may be bonded together directly or by a spacer having a coupling group. This block copolymer is, for example, a biocompatible block copolymer represented by the following formula (1): 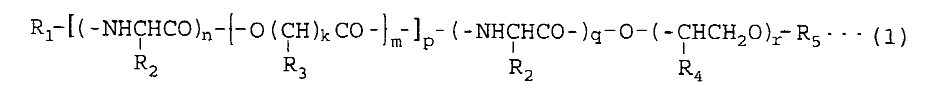 wherein R₁ is a hydrogen atom, alkyl group, substituted alkyl group or protective group for amino group, R₂ is the side chain of natural amino acid or derivative group thereof, R₃ is a hydrogen atom, alkyl group or substituted alkyl group, R₄ is a hydrogen atom or methyl group, R₅ is a hydrogen atom, alkyl group, substituted alkyl group, ligand residue, ligand residue having a coupling group, or group represented by 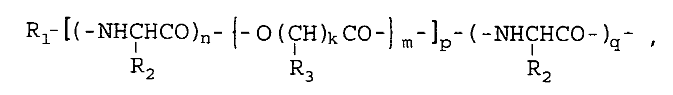 n is an integer of 1 to 20, k is an integer of 1 to 6, m is an integer of 1 to 20, p is an integer of 1 to 20, q is an integer of 0 to 20, and r is an integer of 2 to 870.

### Hydrophobic segment

The hydrophobic segment of the block copolymer used in the present invention is a compound composed of amino acid and hydroxyl carboxylic acid as shown by the above formula (1). The amino acid in use is not particularly limited if it is biodegradable or biocompatible. Not only natural L-α-amino acid but also DL-α-amino acid, D-α-amino acid, synthetic amino acid and derivatives in the side chains of these amino acids may be used. Specific examples of the amino acid include glycine, alanine, valine, leucine, isoleucine, phenyalanine, tyrosine, triptophane, serine, threonine, proline, hydroxyproline, cysteine, methionine, glutamic acid, aspartic acid, lysine, arginine, histidine, hydroxylysine, 4-hydroxyproline, homoproline, norvaline, norleucine, α-t-butylglycine, cyclohexylglycine, asparagine, glutamine and β-cyclohexylalanine. Out of these, glycine, alanine, valine, leucine, isoleucine, phenylalanine, tyrosine, triptophane, glutamic acid, asparagic acid, lysine and arginine are preferred. Further, out of these, alanine, valine, leucine, isoleucine, phenylalanine, glutamic acid, aspartic acid, lysine and arginine are particularly preferred. The hydroxycarboxylic acid in use is not particularly limited if it is biodegradable or biocompatible. Not only α-hydroxycarboxylic acid but also other hydroxycarboxylic acids may be used. Specific examples of the hydroxycarboxylic acid include glycolic acid, lactic acid, hydroxybutyric acid, hydroxyvaleric acid, hydroxycaproic acid and hydroxycapric acid. Out of these, glycolic acid and lactic acid are particularly preferred.

### Depsipeptide

The hydrophobic segment of the block copolymer used in the present invention may be a depsipeptide compound composed of α-amino acid and α-hydroxycarboxylic acid. The α-amino acid in use is not particularly limited if it is biodegradable or biocompatible. Not only natural L-α-amino acid but also DL-α-amino acid, D-α-amino acid and synthetic amino acid may be used. Specific examples of the α-amino acid include α-amino acids and derivatives in the side chains of the α-amino acids out of the above amino acids. The α-hydroxycarboxylic acid in use is not particularly limited if it is biodegradable or biocompatible. Out of the above hydroxycarboxylic acids, glycolic acid and lactic acid which are α-hydroxycarboxylic acids are particularly preferred.

### Hydrolyzability of depsipeptide and possibility of avoiding antigenicity

A depsipeptide has an amide bond and an ester bond in the molecule. Its amide bond portion forms a hydrogen bond between molecules to form a strong intermolecular bond as it has a hydrogen atom whereas its ester bond portion cannot form a hydrogen bond between molecules and is relatively easily hydrolyzable because it does not have a hydrogen atom. Therefore, a depsipeptide having an ester bond in the amide bond chain is readily decomposed in vivo and its decomposition rate can be controlled by the type and number of amino acids and the type and number of hydroxycarboxylic acids and the sequence comprising the amino acids and hydroxycarboxylic acids. Although the antigenicity of a polyamino acid is often controversial, it is considered that a peptide having 5 or less amino acids rarely shows antigenicity. It is possible to carry out molecular design to ensure that a depsipeptide does not show antigenicity. Polydepsipeptides show completely different physical and chemical properties according to the type and the length of sequence of amino acids and the type of hydroxycarboxylic acids constituting each polydepsipeptide. A tetradepsipeptide sequence having 3 amino acids and one hydroxycarboxylic acid completely differs from a tridepsipeptide sequence having two amino acids and one hydroxycarboxylic acid in solvent solubility, the stability of a secondary structure and micelle formability. Further, polydepsipeptides having different properties can be synthesized by changing the type of amino acids and the type of hydroxycarboxylic acids.

A polydepsipeptide having 3 to 5 amino acids and 1 to 3 hydroxycarboxylic acids bonded to these is particularly preferred.

### Number of amino acids, number of hydroxycarboxylic acids and number of repetitions of depsi's

The number (n) of amino acids constituting the polyamino acid of the hydrophobic segment of the block copolymer represented by the formula (1) is an integer of 1 to 20, preferably 1 to 10, more preferably 1 to 6. The number (q) of amino acids constituting the polyamino acid on the hydrophilic segment bonded side of the hydrophobic segment is an integer of 0 to 20, preferably 0 to 10, more preferably 0 to 6. The number (m) of hydroxy acids constituting the polyhydroxycarboxylic acid of the hydrophobic segment is an integer of 1 to 20, preferably 1 to 10, more preferably 1 to 5. The number (p) of repetitions of the amino acid-hydroxycarboxylic acid bonded form is an integer of 1 to 20, preferably 1 to 10, more preferably 1 to 5. The number (k) of the methylene groups and substituted methylene groups of the hydroxycarboxylic acid is an integer of 1 to 6.

When n, p or q is an integer of 2 to 20, R₂'s may be the same or different, when m or p is an integer of 2 to 20, R₃'s may be the same or different, and when r is 2 or more, R₄'s may be the same or different.

### R₁, R₂ and R₃

The terminal R₁ of the hydrophobic segment of the block copolymer represented by the formula (1) is a hydrogen atom, alkyl group, substituted alkyl group or protective group for amino group. Examples of the protective group for amino group include t-butoxycarbonyl group (Boc-), benzyloxycarbonyl group (Z-) and 9-fluorenylmethyloxycarbonyl group (Fmoc-) . It is not particularly limited if it is biocompatible but particularly preferably a t-butoxycarbonyl group (Boc-).

R₂ denotes the side chain of natural amino acid or derivatized side chain (to be referred to as "derivative group" hereinafter), that is, the side chain of natural amino acid or one of its derivative groups for each amino acid unit independently. Examples of the amino acid derivative having a derivative group include glutamic acid 5-derivative, aspartic acid 4-derivative and lysine ε-derivative. Examples of the glutamic acid 5-derivative and aspartic acid 4-derivative include derivatives having a terminal carboxyl group in the side chain protected by a protective group, alkyl esters such as methyl ester and ethyl ester, esters with the hydroxyl group of a drug, amides with the amino group of a drug, and drug bonded forms having a spacer. Examples of the lysine derivative include derivatives having a terminal amino group in the side chain of lysine protected by a protective group, amides with the carboxyl group of a drug, and drug bonded forms having a spacer. Besides the drug derivatives, a 5-ethyl glutamate unit, 4-ethyl aspartate unit and lysine unit having a terminal amino group protected by a benzyloxycarbonyl group are preferably used.

R₃ denotes the side chain of a hydroxycarboxylic acid, that is, a hydrogen atom, alkyl group or substituted alkyl group for each hydroxycarboxylic acid independently. The alkyl group and the substituted alkyl group represented by R₁ and R₃ are each preferably an alkyl group having 1 to 3 carbon atoms or an alkyl group substituted by a substituent. Examples of the alkyl group include methyl group, ethyl group, propyl group and isopropyl group. Examples of the substituent include methyl group, ethyl group and propyl group.

### Hydrophilic segment and R₄

The hydrophilic segment of the block copolymer used in the present invention is composed of polyalkylene oxide, more specifically polyethylene glycol, polyethylene glycol/polypropylene glycol block copolymer, or polypropylene glycol. Polyethylene glycol is more preferred. In the formula (1), the number (r) of repetitions of the alkylene oxide is 2 to 870. It is preferably 3 to 570, more preferably 3 to 250. R₄ is a hydrogen atom or methyl group.

### R₅

R₅ in the above formula (1) is a hydrogen atom, alkyl group, substituted alkyl group, ligand residue, ligand residue having a coupling group or group represented by the formula
R₁-[[-NHCH(R₂)CO-]ₙ-{-O[CH(R₃)]ₖCO}ₘ-]ₚ-[NHCH(R₂)CO-]_{q} (R₁, R₂, R₃, n, k, m, p and q are as defined above) .

Examples of the ligand include lectin, antibody, antibody fragments (such as Fab' fragment), lymphokine, cytokine, receptor proteins (such as CD4, CD8, CD44, CD71, etc.), nucleic acid, antigen, hormone, adhesion factors (such as VCAM-1, ICAM-1, PECAM-1, RGD, NGR, etc) , transferrin, folic acid, growth factors (such as EGF, bFGF, VEGF, etc.) and those specifically binding to a desired target cell.

The block copolymer of the present invention can be manufactured as follows, for example.
(i) An imide ester of amino acid and N-hydroxysuccinic acid having a protected amino group is reacted with hydroxyl groups at both terminals of polyalkylene oxide which is a hydrophilic segment by using dimethyl aminopyridine as a catalyst to obtain polyalkylene oxide in which an amino acid having protected amino groups at both terminals is ester-bonded. The amino protective groups of the amino acid residue at both terminals are treated with hydrochloric acid in dioxane to be removed. By this treatment, polyalkylene oxide from which the protective groups have been removed and whose both terminals have been diaminoacylated or monoaminoacylated polyalkylene oxide in which one of amino acids at both terminals has been eliminated and one hydroxyl group has been regenerated is obtained.
   An imide ester of amino acid and N-hydroxysuccinic acid having a protected amino group is reacted with the amino group of a terminal amino acid to obtain protected dipeptide bonded polyalkylene oxide. An imide ester of an oligodepsipeptide and N-hydroxysuccinic acid having a protected amino group at one terminal and a hydroxycarboxylic acid at the other terminal is reacted with an amino group formed by removing the protective group. When the reaction of this amino group-protected oligodepsipeptide ester is repeated, a block copolymer having a polydepsipeptide hydrophobic segment having an arbitrary sequence of amino acids and an arbitrary sequence of hydroxycarboxylic acids and a polyalkylene oxide hydrophilic segment is obtained.
(ii) A halide of hydroxycarboxylic acid is obtained by acting halogen and halogenated thionyl on hydroxycarboxylic acid, and a depsipeptide is obtained by reacting this with amino acid or polyamino acid in the presence of an alkali. When the amino acid has other functional groups besides two functional groups which are an amino group and a carboxyl group, it is preferred to bond the other functional groups to a protective group during the manufacture of a polydepsipeptide. Then, this amino acid derivative is heated in the presence of an alkali to close the ring of the derivative, thereby making it possible to obtain a cyclic depsipeptide. The cyclic depsipeptide and polyalkylene oxide, monosubstituted polyalkylene oxide or polyalkylene oxide having one protected hydroxyl group are reacted with each other in the presence of a catalyst such as stannous octylate to obtain a block copolymer having a hydrophobic segment composed of amino acid and hydroxycarboxylic acid and a hydrophilic segment composed of polyalkylene oxide.

The hydrophobic segment composed of amino acid and hydroxycarboxylic acid can be synthesized by the method disclosed by JP-A 2004-269462 which was filed by one of the inventors of the present invention. That is, the carboxyl group of amino acid having a protected amino group is reacted with the hydroxyl group of hydroxycarboxylic acid having a non-protected carboxyl group in the presence of an aminopyridine compound catalyst to synthesize a didepsipeptide. The carboxyl group of the obtained didepsipeptide is changed into an imide derivative, the protected amino group of the didepsipeptide is de-protected, and the de-protected amino group of the didepsipeptide is reacted with the carboxyl group of amino acid having a protected amino group to synthesize an oligodepsipeptide.

A reaction for bonding the protected amino acid or protected oligodepsipeptide to polyalkylene oxide may be carried out in a suitable solvent which dissolves these, for example, chloroform, dichloroethane, tetrahydrofuran, acetonitrile or ethyl acetate. After the reaction, the product is dissolved in dichloromethane or chloroform and rinsed with an aqueous solution of sodium hydrogen carbonate or aqueous solution of citric acid to remove the by-product, the solvent is distilled off, and ether is added to precipitate a pure product and isolate it. If necessary, ether is added to a tetrahydrofuran, acetonitrile or ethyl acetate solution of the block copolymer of the present invention to re-precipitate the block copolymer for purification. This purification by re-precipitation is effective for the block copolymer comprising a polydepsipeptide as a hydrophobic segment. In a polypeptide-polyalkylene oxide polymer, a hydrogen bond based on the amide bond of the polypeptide is strongly formed in the molecule, between molecules and between the molecule and the solvent, and it is difficult to find a combination of a solvent and a non-solvent suitable for re-precipitation.

### Micelle and particulate

The block copolymer of the present invention may be used not only to form a micelle containing a drug, a particulate containing a drug and a composite containing a drug but also to manufacture a vesicle such as liposome. According to purpose, the micelle, particulate or composite can be contained in liposome.

The block copolymer of the present invention may be used in combination with a drug. As the drug may be used a physiologically active compound such as natural product, synthetic product, half-synthetic product, fermentation product, biogenic matter or gene engineering product. Synthetic physiologically active compounds, peptides, proteins, hormones, vitamins, enzymes, co-enzymes, fatty acids, fats, genes and derivatives thereof may also be used.

The drug may be insoluble in water, hardly soluble in water, soluble in fat or soluble in water. Examples of the drug include anti-inflammatory drugs (for example, non-steroid drugs such as mefenamic acid, bufexamac and felbinac, and steroid-based drugs such as dexamethazone, prednisolone and beclometasone), defervescents (such as acetaminophen, alclofenac and bufexamac), analgesic drugs (such as aminopyrine, acetaminophen, morphine and buprenorphine), antarthritic/antirheumatic drugs (such as auranofin, azathioprine and methotrexate), antigout drugs (such as allopurinol, probenecid and sulfinpyrazone), cardiac restoratives (such as oxyfedrine and theobromine), antianginal drugs (such as alprenolol, diltiazem, isosorbide dinitrate and nifedipine), beta-adrenergic blocking drugs (such as propranolol hydrochloride, atenolol and carvedilol), calcium antagonist drugs (such as nifedipine, nicardipine and diltiazem), antiarrhythmic drugs (such as quinidine, verapamil and timolol), diuretics (such as furosemide and arbutin), antihypertensives (such as timolol, captopril, nicardipine and prazosin), drugs for improving the disturbances of peripheral circulation (such as prostaglandins), antithrombogenic drugs (such as argatoroban, ozagrel and ticlopidine), antihyperlipemic drugs (such as statin-based drugs, fibrate-based drugs and probucol), antiplatelet drugs (such as prostacyclin), pressor drugs (such as dopamine and norepinephrine), drugs for improving the metabolism of cerebral circulation (such as tiapride hydrochloride and sulpyrine), anticancer drugs (such as doxorubicin, actinomycin, methotrexate, citarabine, cisplatin, paclitaxel and tamoxifen), immunosuppressive drugs (such as azathioprine, tacrolimus hydrate and cyclophosphamide), antiallergic drugs (such as azelastine, cromolyn and pentigetide), antihistamine drugs (such as chlorpheniramine, clemastine and diphenhydramine), bronchodilators (such as albuterol, isoproterenol and ipratropium bromide), antiasthmatic drugs (such as tulobuterol and clenbuterol), antitussive drugs (such as codeine phosphate and dextromethorphan), expectorators (such as ambroxol and carbocysteine), antiulcer drugs (such as cetraxate, ranitidine and cimetidine), antidiarrheals (such as acetyltannic acid and difenoxin), antiemetics (such as difenidol and scopolamine), antidiabetics (such as biguanide-based substances, insulin, tolbutamide and chlorpropamide), hypnotic/sedating drugs (such as nitrazepam, flurazepam and amobarbital), antianxiety drugs (such as diazepam and oxazolam), antipsychotic drugs (such as haloperidol, perphenazine and chlorpromazine), antidepressants (such as dimethazane, imipramine and sulpiride), antivertigo drugs (such as dimenhydrinate, difenidol hydrochloride and betahistine mesylate), antiepilepsy drugs (such as carbamazepine, phenobarbital, 5-hydroxytriptophan), muscle relaxant drugs (such as tubocurarine chloride, baclofen and methocarbamol), antiparkinson drugs(such as amantadine and carbidopa), autonomic effect drugs (such as bethanechol and neostigmine), antibiotics (such as aminoglycoside-based substances, cephalosporin-based substances and macrolide-based substances), antibacterial drugs (such as quinolone-based substances, sulfonamide-based substances and sulfone-based substances), antifungal drugs (such as allylamine-based substances, imidazole-based substances including econazole and triazole-based substances including fluconazole), antivirals (such as acyclovir, cytarabine and amantadine), anthelmintics (such as aminopyrine, niclosamid and quinacrine), hormonal drugs (such as somatostatin, gonadotropin and LHRH), osteoprosis preventing and bone metabolism improving drugs (such as vitamin D, calcitonin and bisphosphonate), vitamin drugs (such as tocophenol and vitamin B12), hemostatics (such as tranexamic acid), enzyme drugs (such as pronase, lysozyme and serrapeptase), vaccines (such as influenza vaccine and cholera vaccine), anesthetics, diagnostic products, contrast drugs and test drugs.

The drug may be either hydrophobic or hydrophilic. The block copolymer of the present invention can form a micelle. There is a case where the hydrophobic segment is existent in the inner core of the micelle and the hydrophilic segment is existent in the outer shell of the micelle. Reversely, there is a case where the hydrophilic segment of the block copolymer is existent in the inner core of the micelle and the hydrophobic segment is existent in the outer shell of the micelle . Further, there is a case where the both segments are existent in a vehicle at random and the micelle does not have a domain structure. It is preferred to select a hydrophobic drug when the hydrophobic segment is existent in the inner core of the micelle and to select a hydrophilic drug when the hydrophilic segment is existent in the inner core of the micelle. When the both segments are existent in the particulate at random, either a hydrophilic or hydrophobic drug may be selected.

Examples of the hydrophobic drug include non-steroid anti-inflammatory drugs (such as indometacin and naproxen), steroid anti-inflammatory drugs (such as dexamethasone, dexamethasone valerate, dexamethasone palmitate, triamcinolone, triamcinolone acetonide, paramethasone acetate, halopredone acetate, hydrocortisone, fludrocortisone acetate, predonisolone butylacetate, predonisolone valerate acetate, predonisolone, betamethasone and betamethasone valerate), anticancer drugs (such as paclitaxel, camptothecin, epotoside, vinblastine, fluorouracil, methotrexate, tegafur, tegafur·uracil, mitomycin C, cisplatin, carboquone, dacarbazine, mercaptopurine, actinomycin D, doxorubicin hydrochloride and tamoxifen citrate), antipyretic analgesics (such as ibuprofen and buprenorphine), antiasthma drugs (such as azelastine, ketotifen and fluticasone propionate), antidepressants (such as oxypertine and nefopam), antigout drugs (such as allopurinol, colchicine, sulfinpyrazone and probenecid), CNS drugs (such as aniracetam), antihypertensives (such as captopril, enalapril and manidipine hydrochloride), antiplatelet drugs (such as prostaglandins), antihyperlipemic drugs (such as clofibrate and gamma orizanol), brochodilators (such as theophyline, methoxyphenamine hydrochloride and tulobuterol hydrochloride), antiallergic drugs (such as emedastine difumarate, tranilast and terfenadine), antienuresis drug (such as gestoronone caproate), antiviral drugs (such as acyclovir and ribavirin)and fluorescent dyes (such as pyrene-based fluorescent dyes and rhodamine-based fluorescent dyes).

A water-soluble polypeptide is used as the hydrophilic drug. Examples of the water-soluble polypeptide include cytokine, hematopoietic factors, growth factors and enzymes. Out of these, cytokine is preferred, as exemplified by lymphokine (such as interferon α, interferon β, interferon γ, interleukins (IL-2 to IL-12) andmonokines (interleukin-1, tumor necrosis factors (TNF)).

In addition, drugs such as luteinzing hormone release hormones and derivatives or analogues, insulin and derivatives or analogues, somatostatin and derivatives or analogues, growth hormones, growth hormone release hormones, prolactin, erythropoietin, adrenal cortex hormones and derivatives or analogues, melanocyte stimulating hormones, thyroid hormone release hormones and derivatives or analogues, thyroid stimulating hormones, luteinzing hormones, follicle-stimulating hormones, vasopressin and derivatives, oxytocin, calcitonin and derivatives or analogues, glucagon, gastrin, secretin, pancreozymin, cholecystokinin, angiotensin, human placental lactogen, human chorionic gonadotropin, enkephalin and derivatives or analogues, endorphin, kyotorphin, tuftsin, thymopoietin, thymosin, thymic humoral factors, serum thymic factors and derivatives thereof, colony inducing factors (such as CSF, GCSF, GMCSF, MCSF, etc.), motilin, deinorphine, bombegin, neurotensin, caerulein, bradykinin, atrial natriuretic factors, nerve growth factors, cell growth factors (such as EGF, TGF-β, PDGF, acid FGF, basic FGF, etc.), neurotrophic factors (such as NT-3, NT-4, CNTF, GDNF, BDNF, etc.), peptides having an endothelin antagonistic effect and analogues thereof may be used.

The drug used in the present invention may be as it is as a matter of course or may be in a pharmacologically allowable form. When the drug has a basic group such as an amino group, examples of the salt of the drug include salts comprising an inorganic acid (for example, hydrochloric acid, sulfuric acid, nitric acid or boric acid) and salts comprising an organic acid (for example, carbonic acid, bicarbonic acid, succinic acid, acetic acid, propionic acid, fumaric acid or trifluoroacetic acid). When the drug has a acidic group such as carboxyl group, examples of the salt of the drug include salts comprising an inorganic base (for example, an alkali metal such as sodium or potassium, or an alkaline earth metal such as calcium or magnesium) and salts comprising an organic base (for example, an organic amine such as triethylamine or a basic amino acid such as arginine) . The drug may form a metal complex compound such as copper complex or zinc complex.

To prepare a drug-containing micelle comprising the block copolymer of the present invention as a constituent component, thin film underwater stirring, solution underwater diluting and dialysis methods are employed. They may be used alone or in combination.
(1) thin film underwater stirring method
   At least one type of block copolymer having micelle forming ability out of the block copolymers of the present invention and a drug are dissolved in a solvent, the resulting solution is put into a vessel, and the solvent is removed to form a thin film. Water is added to the thin film and stirred to form a micelle. The solvent used herein is not particularly limited if it can dissolve the block copolymer and the drug. Examples of the solvent include tetrahydrofuran, dioxane, acetone, methyl ethyl ketone, acetonitrile, dimethyl sulfoxide, methanol, ethanol, benzene, toluene, dimethyl acetamide and dimethyl formamide. They may be used alone or in combination. Another solvent may be added to the above solvent to such an extent that the block copolymer is not precipitated. Additives may be added to a water phase. The additives include a pH control agent (for example, phosphate buffer, carbonate buffer, acetate buffer, diluted hydrochloric acid, sodium hydroxide, etc.), anionic surfactant, nonionic surfactant, emulsifying agent such as polyoxyethylene castor oil derivative, polyethylene glycol, polyvinyl pyrrolidone, polyvinyl alcohol, carboxymethyl cellulose, lecithin, gelatin or hyaluronic acid, and sugar such as mannitol. The preparation temperature is preferably about 0 to about 80°C, more preferably about 5 to about 50°C. Further, external shear force or ultrasonic irradiation may be applied at the time of underwater agitation, or the residual solvent can be removed by an evaporator under reduced pressure in accordance with a commonly used method. Examples of the apparatus for applying external shear force include a turbine stirrer and homogenizer.
(2) solution underwater diluting method At least one type of block copolymer having micelle forming ability out of the block copolymers of the present invention and a drug are dissolved in a solvent, the resulting solution is added to water little by little under agitation, and the solvent is removed into water to form a polymer micelle. The solvent used herein is not particularly limited if it is miscible with water. Examples of the solvent include methanol, ethanol, dimethyl acetamide, dimethyl formamide, dimethyl sulfoxide, tetrahydrofuran, dioxane, acetone, acetonitrile and mixed solutions of these and water. The solvents may be used alone or in combination of two or more. Out of these, a combination of tetrahydrofuran, acetone and water is preferred. Since a good solvent for the block copolymer changes by the composition of the polymer as a matter of course, it is preferred to select a solvent according to the polymer in use. The same additives as those which are added to the water phase in the above thin film underwater stirring method may also be added to a water phase. The preparation temperature is preferably about 0 to about 80°C, more preferably about 5 to about 50°C. Further, external shear force or ultrasonic irradiation may be applied at the time of underwater agitation, or the residual solvent can be removed by an evaporator under reduced pressure in accordance with a commonly used method or by an aeration process. Examples of the apparatus for applying external shear force include a turbine stirrer and homogenizer.
(3) dialysis method
   At least one type of block copolymer having micelle forming ability out of the block copolymers of the present invention and a drug are dissolved in a solvent, the resulting solution is put into a dialysis tube to be dialyzed in water, and the solvent is removed to form a micelle. The solvent used herein is not particularly limited if it can dissolve the block copolymer and is miscible with water. Examples of the solvent include methanol, ethanol, dimethyl acetamide, dimethyl formamide, dimethyl sulfoxide, tetrahydrofuran, dioxane, acetone and acetonitrile. They may be used alone or in combination of two or more. Another solvent may be added to the above solvent to such an extent that the block copolymer and the drug are not precipitated. The ratio of the block copolymer to the solvent is not particularly limited. A dialysis membrane having a suitable molecular weight fractionation size may be selected according to the molecular weight of the block copolymer in use. Although a cellulose membrane is often used, the present invention is not limited to this. Additives may be added to a dialysis solution. The additives include a pH control agent (for example, phosphate buffer, carbonate buffer, acetate buffer, diluted hydrochloric acid, sodium hydroxide, etc.), anionic surfactant, nonionic surfactant, emulsifying agent such as polyoxyethylene castor oil derivative, polyethylene glycol, polyvinyl pyrrolidone, polyvinyl alcohol, carboxymethyl cellulose, lecithin, gelatin or hyaluronic acid, and sugar such as mannitol. Although the dialysis time is generally about 5 minutes to about 24 hours each time, a micelle is prepared by repeating the operation of exchanging the liquid of an external water phase one or more times. The preparation temperature is preferably about 0 to about 80°C, more preferably about 5 to about 50°C. The micelle prepared by this can be obtained in a transparent or slightly clouded state in the dialysis membrane.

In the present invention, the constitution of a micelle which can be formed from the block copolymer having hydrophobic and hydrophilic segments can be changed by the preparation method. When the micelle is prepared in a large amount of an aqueous solution after the block copolymer is dissolved or dispersed into an organic solvent, it becomes an O/W type micelle having a hydrophobic segment in the inner core and a hydrophilic segment in the outer shell. When the micelle is prepared in a large amount of an organic solvent after the block copolymer is dissolved or dispersed into an aqueous solution, it becomes a W/O type micelle having a hydrophilic segment in the inner core and a hydrophobic segment in the outer shell. In general, a core-shell type micelle having a hydrophobic segment in the inner core and a hydrophilic segment in the outer shell is obtained. In this case, it is considered that the hydrophobic segments of the block copolymers gather and agglomerate together at a high density to form a core and that the hydrophilic segments extend outward from the surface of the particle. When the terminals of the hydrophilic segments are protected or modified, the existence of the partial agglomeration of the hydrophilic segments is conceivable. These preparing methods can be changed according to purpose. For example, to encapsulate the drug, the method is determined by the physical properties such as solubility in water or an organic solvent and stability of the drug.

The average particle diameter of the drug-containing micelles or the agglomerate thereof obtained by the above method is preferably 1 to 1,000 nm, more preferably 5 to 500 nm, particularly preferably 5 to 300 nm when they are dispersed in water. The factors of determining the particle diameters of these are the composition of the block copolymer and the preparation method of the particle. Since the particle diameter can be changed by application purpose, it is not limited to the above range. The particle diameter can be measured by a known method, for example, a light scattering measuring method or microscopic observation.

In all of the above preparation methods, after the formation of a drug-containing micelle, it is freeze dried or vacuum dried while it is dispersed in water and can be stored as a dry powder. An additive may be added to suppress agglomeration during drying operation. Examples of the additive include mannitol, lactose, glucose, trehalose, starches, hyaluronic acid, alkali metal salts thereof, water-soluble polysaccharides, polyethylene glycol, proteins such as glycine, fibrin or collagen, sodium chloride, and inorganic salts such as sodium hydrogen phosphate. The amount of the additive is not particularly limited if agglomeration can be suppressed when the micelle is re-dispersed.

The block copolymer of the present invention in which a ligand is bonded to the hydrophilic segment has targeting ability in vivo. Since the polymer micelle based on the present invention can be prepared under relatively mild conditions, it is suitable for the maintenance of the activity of the ligand and the encapsulation of the unstable drug. Since the particle diameter can be controlled by suitably changing the molecular weights of the hydrophobic segment and the hydrophilic segment, the residence in the blood of a preparation containing the drug can also be controlled by adjusting the particle diameter. The block copolymer of the present invention can be used for the solubilization in water of a hydrophobic compound as understood from the above performance.

Since the polymer micelle is generally a preparation having a hydrophobic segment as a core, the drug to be encapsulated is preferably hydrophobic. However, even a water-soluble compound having a hydrophobic portion can form a micelle together with the block copolymer in the hydrophobic portion. A drug compound which can reinforce the efficacy of the drug by directing it to a specific organ (such as kidney, liver, lung, pancreas, brain, etc.) or a damaged site or organ (such as cancer or inflamed site) or by extending its residence in the blood is preferred.

The block copolymer of the present invention enables the drug to be encapsulated in the inner core composed of the hydrophobic segment of the micelle. The method of encapsulating the drug of the present invention enables the preparation of a polymer micelle having the drug and the hydrophobic segment as the core and the hydrophilic segment on the surface by adding the drug to a polymer solution. To trap the drug efficiently, a suitable solvent may be added. For example, it is conceivable that the drug is encapsulated by adding a solvent such as chloroform or dichloromethane so as to stabilize a hydrophobic environment in the block copolymer. Further, it is also conceivable that the block copolymer is used to prepare a polymer micelle and then the drug dissolved in a solvent is added to the polymer micelle solution to impregnate the core portion of the polymer micelle with the drug. In this case, the impregnation efficiency can be increased by suitably changing the pH of the solution or the concentration of the salt. It is further conceivable that the block copolymer is used to prepare a polymer micelle and the drug is added to and kneaded with the polymer micelle solution to impregnate the core portion of the polymer micelle with the drug. The hydrophobic drug to be encapsulated is the same as above. Unlike the above polymer micelle, a polymer micelle having a hydrophilic segment in the core and a hydrophobic segment in the shell is also preferred and obtained by encapsulating a hydrophilic drug in the core of the polymer micelle and shows sustained-release properties and drug stability. The hydrophilic drug is the same as above. This polymer micelle can be administered as will be described hereinafter.

To prepare a drug-containing particulate comprising the block copolymer of the present invention as a constituent component, underwater drying, ultrasonic treating and freeze drying methods are employed. They may be used alone or in combination.
(1) underwater drying method
   At least one type of block copolymer having particulate forming ability out of the block copolymers of the present invention and a drug are dissolved in a solvent, the resulting solution is added to water little by little under agitation, and the solvent is removed or diffused to form a particulate. The solvent used herein is not particularly limited if it can dissolve the block copolymer and the drug. Examples of the solvent include methanol, ethanol, benzene, toluene, dimethyl acetamide, dimethyl formamide, dimethyl sulfoxide, tetrahydrofuran, dioxane, acetone, acetonitrile and mixed solutions of these and water. They may be used alone or in combination of two or more. The same additives as those which are added to the water phase in the above thin film underwater stirring method may also be added to a water phase. The amount of water for an organic solvent may be selected arbitrarily. The preparation temperature is preferably about 0 to about 80°C, more preferably about 5 to about 50°C. Further, external shear force can be applied at the time of underwater drying, or the solvent can be removed by aeration or an evaporator under reduced pressure. Examples of the apparatus for applying external shear force include a turbine stirrer and homogenizer.
(2) ultrasonic treating method
   At least one type of block copolymer having particulate forming ability out of the block copolymers of the present invention and a drug are dissolved in a suitable solvent, and the resulting solution is dispersed in a large amount of water. Ultrasonic irradiation is carried out at 1 W to about 200 W, preferably 1 W to about 10 W for preferably 1 second to about 24 hours, more preferably 1 minute to about 5 hours although these conditions cannot be determined unconditionally and change according to the shape and throughput of the apparatus. The preparation temperature is about 0 to about 80°C, preferably about 5 to about 50°C. This method is often carried out in combination with the above underwater drying method.

The content of the block copolymer in the particulate of the present invention is generally 10 to 100 wt%, preferably 30 to 100 wt% based on the particulate. When the drug is contained in the particulate of the present invention, the content of the drug in the particulate of the present invention is determined by the amount required for medical treatment and not particularly limited. The particulate of the present invention may be mixed with a dispersant (surfactant such as polysorbate 80 or polyoxyethylene curable castor oil 60; polysaccharide such as carboxymethyl cellulose, sodium alginate or sodium hyaluronate; protamine sulfate; polyethylene glycol 400), preserving agent (such as methyl paraoxybenzoate or propyl paraoxybenzoate), tonicity agent (such as sodium chloride, mannitol, sorbitol or glucose), oil and fat (such as sesame oil or corn oil), phosphatide (such as lecithin), vehicle (such as lactic acid, corn starch, mannitol or cellulose) binder (such as lactose, gum arabic, methyl cellulose, carboxymethyl cellulose or dextrin), and disintegrant (such as carboxymethyl cellulose calcium).

To prepare a drug composite particle comprising the block copolymer of the present invention as a constituent component, for example, an underwater kneading method is employed. A drug composite can be manufactured by adding a drug to an aqueous solution of the block copolymer and kneading them together. A composite of a nano-order drug crystal and the block copolymer can be formed by carrying out kneading efficiently and the suspended state of the drug is kept stably.

The micelle, particulate or composite containing a drug of the present invention can be formulated into various forms by adding various medicinal additives and administered as an injectable or implantable preparation (for example, intravenous, subcutaneous, intramuscular, intradermal, indirect intracavitary or interstitial administration), transmucosal preparation (such as oral cavity mucosal, nasal, suppository, vaginal or pulmonary preparation), transdermal preparation (such as ointment, cream or gel) or oral preparation (such as tablet, capsule or granule).

To formulate an injectable or implantable preparation of the micelle or particulate containing a drug of the present invention, an aqueous preparation or aqueous suspension of the micelle or particulate containing a surfactant (such as polysorbate or polyoxyethylene hydrogenated castor oil), dispersant (such as carboxymethyl cellulose, sodium alginate, carboxyvinyl polymer, polyethylene glycol or sodium hyaluronate), preserving agent (such as methyl paraoxybenzoate or propyl paraoxybenzoate), tonicity agent (such as sodium chloride, mannitol, sorbitol, glucose, proline) and buffer (such as sodium phosphate or sodium hydrogen phosphate), or an oil suspension obtained by dispersing the micelle or particulate together with vegetable oil such as sesame oil or vegetable oil may be prepared. The aqueous preparation and the aqueous suspension may be freeze-dried to obtain a freeze-dried preparation.

To formulate an aseptic preparation of the micelle or particulate containing a drug of the present invention, a method comprising aseptic filtration and aseptic filling steps, a method making use of gamma sterilization, a method comprising adding an antiseptic and a method comprising the sterilization of all the manufacturing steps are employed but the present invention is not limited to these.

The micelle or particulate containing a drug of the present invention may be used as a safe medicine for mammals. The applied dose of the micelle or particulate containing a drug of the present invention which differs according to the type and content of the drug as the principal drug, the form of the preparation, administration route, target disease and target animal may be the effective amount of the drug. The number of doses is one or several per day, one per week, one every two weeks, one per month or one every several months and suitably selected according to the type and content of the drug as the principal drug, the form of the preparation, administration route, target disease or target animal.

As described above, the block copolymer of the present invention can form a micelle, particulate or composite together with a hydrophobic compound. A micelle of the block copolymer can solubilize the hydrophobic compound and enables the intravenous administration of the compound. The accumulation on an affected site of the micelle by a ligand is possible. Further, since the block copolymer is biocompatible and has sustained-release properties, the sustention of drug efficacy can be expected. The micelle can be expected to improve oral absorption. The particulate can be expected to release the drug for a long time as a hypodermic injection, and the composite makes it possible to prepare a nano-particle of a hardly absorbable drug and can be expected to improve oral absorption. The block copolymer of the present invention can provide various characteristic properties such as selectivity by changing the type and number of constituent amino acids, the type and number of hydroxycarboxylic acids, the rule of the sequence or the modifying group.

### Examples

The following examples are provided for the purpose of further illustrating the present invention but are in no way to be taken as limiting.

### Reference Example 1

### Synthesis of aminoacyl polyethylene glycol

200 g (0.05 mol) of polyethylene glycol having an average molecular weight of 4,000 (HO-PEG₄₀₀₀-OH) was dissolved in 400 ml of acetonitrile. 55 g (0.15 mol) of t-butoxycarbonyl-L-phenylalanine-N-hydroxysuccinimide ester (Boc-Phe-ONSu) and 2.65 g (0.015 mol) of 4-dimethylaminopyridine were added to the resulting solution and stirred at room temperature for 2 days. After the end of a reaction, the solvent was distilled off under reduced pressure, and the residue was dissolved in 600 ml of dichloromethane and rinsed with a 10 % aqueous solution of citric acid, a saturated aqueous solution of sodium hydrogen carbonate and saturated saline. The solution was dried on anhydrous sodium sulfate and concentrated, and ether was added to the residue to crystallize it. The obtained crude product was dissolved in acetonitrile, the resulting solution was filtered with a 450 nm membrane filter, and the filtrate was concentrated under reduced pressure. Ether was added to the residue to recrystallize it. The NMR spectrum of the recrystallized product showed that it was Boc-Phe-O-PEG₄₀₀₀-O-Phe-Boc in which Boc-Phe was ester-bonded to both terminals of HO-PEG₄₀₀₀-OH. The yield was 215 g (94 %) . When 90 g (0.02 mol) of Boc-Phe-O-PEG₄₀₀₀-O-Phe-Boc was treated with 4M-HC1 in dioxane and the solvent was distilled off under reduced pressure, the solid residue was obtained. Ether was added to this residue, and the obtained crystal was filtered. The NMR spectrum of this crystal showed that it was HCl·-H-Phe-O-PEG-OH. The yield of this crystal was 77.8 g (93 %) .

### Reference Example 2

### Introduction of t-butoxycarbonyl-L-leucine into aminoacyl polyethylene glycol

38.9 g (0.01 mol) of the above polyethylene glycol-L-phenyl alaninate hydrochloride was dissolved in 300 ml of a mixed solvent of acetonitrile (ACN) and tetrahydrofuran (THF) in a weight ratio of 1:1, 1.15 ml (0.0105 mol) of N-methylmorpholine (NMM) was added to the resulting solution, and 4.92 g (0.015 mol) of t-butoxycarbonyl-L-leucine-N-hydroxysuccinimide ester (Boc-Leu-ONSu) was added to the solution and stirred at room temperature for one night. After the end of a reaction, the solution was concentrated under reduced pressure, the residue was dissolved in dichloromethane, the resulting solution was rinsed with a 10 % aqueous solution of sodium hydrogen carbonate, a saturated aqueous solution of citric acid and water and dried on anhydrous sodium sulfate. The obtained solution was concentrated under reduced pressure, and ether was added to the residue to crystallize it.

The obtained Boc-Leu-Phe-O-PEG₄₀₀₀-OH was dissolved in THF, and ether was added to recrystallize it. The yield was 41 g (94 %).

### Reference Example 3

### Synthesis of t-butoxycarbonyl-L-leucyl-L-leucyl-L-alanyl-L-lactate-N-hydroxysuccinimide ester (Boc-Leu-Leu-Ala-Lac-ONSu)

45 g (0.5 mol) of L-lactic acid was dissolved in 500 ml of THF, and 0.5 ml (0.5 mol) of pyridine was added. 143 g (0.5 mol) of Boc-L-alanine-ONSu was added at room temperature under agitation, and 6.1 g (0.05 mol) of 4-dimethylaminopyridine was further added. They were stirred at room temperature for 1.5 days, and the solvent was distilled off under reduced pressure. The residue was dissolved in 500 ml of ethyl acetate, and the resulting solution was rinsed with a saturated aqueous solution of citric acid and water. The solution was extracted with a 10 % aqueous solution of sodium hydrogen carbonate 3 times, and the obtained extracts were combined together and made acidic with citric acid. The acidic aqueous solution was extracted with 300 ml of ethyl acetate three times. The combined extract solution was rinsed with water and dried on anhydrous sodium sulfate. When the solvent was distilled off under reduced pressure and hexane was added to the obtained oil, the oil crystallized. It was recrystallized with ethyl acetate to obtain pure Boc-L-alanyl-L-lactic acid (Boc-Ala-Lac-OH). The yield was 91 g (7 %). This didepsipeptide was treated with 4M hydrochloric acid in dioxane to remove the Boc protective group. The obtained didepsipeptide hydrochloride was dissolved in 400 ml of THF, and 28 ml of pyridine was added under agitation. 121 g (0.37 mol) of Boc-Leu-ONSu was added to this solution, and 38.5 ml (0.35 mol) of NMM was added under agitation. They were stirred at room temperature for one night. This solution was concentrated under reduced pressure, and the residue was dissolved in 500 ml of ethyl acetate. This solution was treated as described above to obtain 115 g (88 %) of Boc-Leu-Ala-Lac-OH. The Boc group of this tridepsipeptide was removed, and the obtained product was reacted with Boc-Leu-ONSu to obtain 127 g (85 %) of Boc-Leu-Leu-Ala-Lac-OH. This Boc-tetradepsipeptide was dissolved in 400 ml of THF, and 34.5 g (0.3 mol) of N-hydroxysuccinimide (HOSu) was added. The resulting solution was cooled to -10°C, and 55.4 g (0.27 mol) of dicyclohexyl carbodiimide was added, stirred at -10°C for 3 hours and at room temperature for one night. The solution was diluted with 500 ml of ethyl acetate, the formed dicyclohexyl urea crystal was filtered off, and the filtrate was concentrated under reduced pressure. Hexane was added to the residue to crystallize it. The crystallized product was recrystallized with ethyl acetate to obtain 138 g (90 %) of pure Boc-Leu-Leu-Ala-Lac-ONSu.

### Example 1

### Manufacture of Boc-Leu-Leu-Ala-Lac-Leu-Phe-O-PEG₄₀₀₀-OH

40 g (9.2 mmol) of Boc-Leu-Phe-O-PEG₄₀₀₀-OH was treated with 4M hydrochloric acid/dioxane to remove the Boc protective group. The obtained hydrochloride was dissolved in ACN-THF (1:1) , and 1 ml of NMM was added. 8.1 g (13.9 mmol) of t-butoxycarbonyl-L-leucyl-L-leucyl-L-alanyl-L-lactate-N-hydroxysuccinimide ester (Boc-Leu-Leu-Ala-Lac-ONSu) which is an N-protected tetradepsipeptide active ester was added to this resulting solution and stirred at room temperature for one night. The solution was concentrated under reduced pressure, and the residue was dissolved in dichloromethane, rinsed with a 10 % aqueous solution of sodium hydrogen carbonate, a saturated aqueous solution of citric acid and water, and dried on anhydrous sodium sulfate. The solution was concentrated under reduced pressure, and ether was added to the residue to crystallize it. The obtained crystal was recrystallized with THF-ether to obtain 41.8 g (96 %) of pure Boc-Leu-Leu-Ala -Lac -Leu- Phe-O-PEG₄₀₀₀-OH.

### Example 2

### Manufacture of Boc- (Leu-Leu-Ala-Lac) ₂-Leu-Phe-0-PEG₄₀₀₀-OH

When the Boc protective group of Boc-Leu-Leu-Ala-Lac-Leu-Phe-O-PEG₄₀₀₀-OH of Example 1 was removed by a treatment with 4M-hydrochloric acid/dioxane and the obtained product was reacted with Boc-Leu-Leu-Ala-Lac-ONSu by the same operation under the same conditions as in Example 1, Boc- (Leu-Leu-Ala-Lac)₂-Leu-Phe-O-PEG₄₀₀₀-OH was obtained at a yield of 92 %.

### Example 3

### Manufacture of Boc-(Leu-Leu-Ala-Lac)₃-Leu-Phe-0-PEG₄₀₀₀-OH

When the Boc protective group of
Boc- (Leu-Leu-Ala-Lac)₂-Leu-Phe-O-PEG₄₀₀₀-OH of Example 2 was removed by a treatment with 4M-hydrochloric acid/dioxane and the obtained product was reacted with
Boc-Leu-Leu-Ala-Lac-ONSu by the same operation under the same conditions as in Example 1,
Boc-(Leu-Leu-Ala-Lac)₃-Leu-Phe-O-PEG₄₀₀₀-OH was obtained at a yield of 92 %.

The specific optical rotation of this product was [α]_{D}²⁵ = -13.1 (c = 1.0, CH₃CN) and the NMR spectrum [0.84 ppm (42H) multiple peaks based on two CH₃'s of Leu; 1.2 to 1.7ppm (48H) multiple peaks based on β-CH₃ of Ala and Lac, β-CH₂ of Leu, γCH and 3 CH₃'s of Boc; 3.5 ppm (360H) single peak based on CH₂ of PEG; 4.2 to 4.9 ppm (14H) multiple peaks based on α-CH of amino acid and lactic acid; 7.2 ppm (5H) multiple peaks based on aromatic ring hydrogen of Phe; 7.6 to 8.5 ppm (11H) multiple peaks based on NH of amide bond] showed that this was Boc- (Leu-Leu-Ala-Lac)₃-Leu-Phe-O-PEG₄₀₀₀-OH.

### Example 4

### Manufacture of Boc-(Leu-Leu-Ala-Lac)₄-Leu-Phe-O-PEG₄₀₀₀-OH

When the Boc protective group of
Boc- (Leu-Leu-Ala-Lac) ₃-Leu-Phe-0-PEG₄₀₀₀-OH of Example 3 was removed by a treatment with 4M-hydrochloric acid/dioxane and the obtained product was reacted with
Boc-Leu-Leu-Ala-Lac-ONSu by the same operation under the same conditions as in Example 1,
Boc- (Leu-Leu-Ala-Lac)₄-Leu-Phe-O-PEG₄₀₀₀-OH was obtained at a yield of 95 %.

### Example 5

### Manufacture of Boc-(Leu-Leu-Ala-Lac)₅-Leu-Phe-O-PEG₄₀₀₀-OH

When the Boc protective group of Boc- (Leu-Leu-Ala-Lac)₄-Leu-Phe-O-PEG₄₀₀₀-OH of Example 4 was removed by a treatment with 4M-hydrochloric acid/dioxane and the obtained product was reacted with
Boc-Leu-Leu-Ala-Lac-ONSu by the same operation under the same conditions as in Example 1,
Boc- (Leu-Leu-Ala-Lac) ₅-Leu-Phe-O-PEG₄₀₀₀-OH was obtained at a yield of 95 %.

The specific optical rotation of this product was [α]_{D}²⁵ = -13.7 (c = 1.0, CH₃CN) and the NMR spectrum [0.84 ppm (66H) multiple peaks based on two CH₃' s of Leu; 1.2 to 1. 7ppm (72H) multiple peaks based on β-CH₃ of Ala and Lac, -CH₂ of Leu, γCH and 3 CH₃'s of Boc; 3.5 ppm (360H) single peak based on CH₂ of PEG; 4.2 to 4.9 ppm (22H) multiple peaks based on α-CH of amino acid and lactic acid; 7.2 ppm (5H) multiple peaks based on aromatic ring hydrogen of Phe; 7.6 to 8.5 ppm (17H) multiple peaks based on NH of amide bond] showed that this was Boc- (Leu-Leu-Ala-Lac) ₅-Leu-Phe-O-PEG₄₀₀₀-OH.

### Example 6

### Manufacture of Boc-Leu-Leu-Ala-Lac-Leu-Phe-O-PEG₆₀₀-O-Phe-Leu-Lac-Ala-Le u-Leu-Boc

Boc-Leu-Leu-Ala-Lac-Leu-Phe-O-PEG₆₀₀-O-Phe-Leu-Lac-Ala-Leu-Leu-Boc was obtained at a yield of 96 % by using polyethylene glycol having an average molecular weight of 600 in place of polyethylene glycol having an average molecular weight of 4,000 in Reference Example 1 and treating it in the same manner as in Reference Example 2 and Example 1.

### Example 7

### Manufacture of Boc-Leu-Leu-Ala-Lac-Leu-Phe-O-PEG₁₅₄₀-O-Phe-Leu-Lac-Ala-L eu-Leu-Boc

Boc-Leu-Leu-Ala-Lac-Leu-Phe-O-PEG₁₅₄₀-O-Phe-Leu-Lac-Ala-Leu-Leu-Boc was obtained at a yield of 95 % by using polyethylene glycol having an average molecular weight of 1,540 in place of polyethylene glycol having an average molecular weight of 4,000 in Reference Example 1 and treating it in the same manner as in Reference Example 2 and Example 1.

### Example 8

### Manufacture of Boc-(Leu-Leu-Ala-Lac)₅-Leu-Phe-0-PEG₆₀₀0-OH

Boc-(Leu-Leu-Ala-Lac)₅-Leu-Phe-O-PEG₆₀₀₀-OH was obtained at a yield of 95 % by using polyethylene glycol having an average molecular weight of 6, 000 in place of polyethylene glycol having an average molecular weight of 4,000 in Reference Example 1 and treating it in the same manner as in Reference Example 2 and Examples 1 to 5.

### Example 9

### Manufacture of Boc-(Leu-Leu-Ala-Lac)₅-Leu-Phe-O-PEG₂₀₀₀₀-OH

Boc-(Leu-Leu-Ala-Lac)₅-Leu-Phe-O-PEG₂₀₀₀₀-OH was obtained at a yield of 97 % by using polyethylene glycol having an average molecular weight of 20,000 in place of polyethylene glycol having an average molecular weight of 4, 000 in Reference Example 1 and treating it in the same manner as in Reference Example 2 and Examples 1 to 5.

### Example 10

### Manufacture of Boc- (Leu-Leu-Ala-Lac)₅-Leu-Phe-O-PEG₄₀₀₀-OCH₃

200 g (0.05 mol) of monomethoxy polyethylene glycol having an average molecular weight of 4, 000 (HO-PEG₄₀₀₀-OCH₃) was dissolved in 400 ml of acetonitrile, and 55 g (0.15 mol) of t-butoxycarbonyl-L-phenylalanine-N-hydroxysuccinimide ester (Boc-Phe-ONSu) and 2.65 g (0.015 mol) of 4-dimethylaminopyridine were added to this solution and reacted at room temperature for 2 days. After the end of the reaction, the solvent was distilled off under reduced pressure, and the residue was dissolved in 600 ml of dichloromethane and rinsed with a 10 % aqueous solution of citric acid, a saturated aqueous solution of sodium hydrogen carbonate and saturated saline. The solution was dried on anhydrous sodium sulfate and concentrated, and ether was added to the residue to crystallize it. The obtained crude product was dissolved in acetonitrile, the resulting solution was filtered with a 450 nm membrane filter, and the filtrate was concentrated under reduced pressure. Ether was added to the residue to recrystallize it. The NMR spectrum of this crystal showed that this was Boc-Phe-O-PEG₄₀₀₀-OCH₃ in which Boc-Phe was ester bonded to the terminal of HO-PEG₄₀₀₀-OCH₃. The yield was 200 g (94 %) . When 90 g (0.02 mol) of Boc-Phe-O-PEG₄₀₀₀-OCH₃was treated with 4M-HCl in dioxane and the solvent was distilled off under reduced pressure, the solid residue was obtained. Ether was added to the residue, and the obtained crystal was filtered. The NMR spectrum showed that this crystal was HCl. H-Phe-O-PEG-OCH₃. The yield was 77.8 g (93 %). This product was treated in the same manner as in Reference Example 2 and Examples 1 to 5 to obtain Boc-(Leu-Leu-Ala-Lac)₅-Leu-Phe-O-PEG₄₀₀₀-OCH₃

### Example 11

### Manufacture of Boc-Leu-Leu-Ala-Hea-Ala-Phe-O-PEG₄₀₀₀-OH

38.9 g (0.01 mol) of polyethylene glycol L-phenylalaninate hydrochloride synthesized in Reference Example 1 was dissolved in 300 ml of a mixed solvent of acetonitrile (ACN) and tetrahydrofuran (THF) in a weight ratio of 1:1, 1.15 ml (0.0105 mol) of NMM was added to the resulting solution, and 4.3 g (0.015 mol) of t-butoxycarbonyl-L-alanine-N-hydroxysuccinimide ester (Boc-Ala-ONSu) was added to the resulting solution and stirred at room temperature for one night. After the end of a reaction, the solution was concentrated under reduced pressure, the residue was dissolved in dichloromethane, and the solution was rinsed with a 10 % aqueous solution of sodium hydrogen carbonate, a saturated aqueous solution of citric acid and water and dried on anhydrous sodium sulfate. The solution was concentrated under reduced pressure, and ether was added to the residue to crystallize it. The obtained Boc-Ala-Phe-O-PEG₄₀₀₀-OH was dissolved in THF, and ether was added to the above product to recrystallize it. The yield was 39 g (90 %) . 43.2 g (10 mmol) of Boc-Ala-Phe-O-PEG₄₀₀₀-OH was treated with 4M-hydrochloric acid/dioxane to remove the Boc protective group. The obtained hydrochloride was dissolved in ACN-THF (1:1) , and 1 ml of NMM was added. 8.55 g (15 mmol) of t-butoxycarbonyl-L-leucyl-L-leucyl-L-alanyl-L-glycolate-N-hydroxysuccinimide ester (Boc-Leu-Leu-Ala-Hea-ONSu) which is an N-protected tetradepsipeptide active ester was added to the resulting solution and stirred at room temperature for one night. The solution was concentrated under reduced pressure, and the residue was dissolved in dichloromethane, rinsed with a 10 % aqueous solution of sodium hydrogen carbonate, a saturated aqueous solution of citric acid and water, and dried on anhydrous sodium sulfate. The solution was concentrated under reduced pressure, and ether was added to the residue to crystallize it. The obtained crystal was recrystallized with THF-ether to obtain 42.1 (90 %) of pure
Boc-Leu-Leu-Ala-Hea-Ala-Phe-O-PEG₄₀₀₀-OH.

### Example 12

### Manufacture of Boc- (Leu-Leu-Ala-Hea)₂-Ala-Phe-O-PEG₄₀₀₀-OH

When the Boc protective group of Boc-Leu-Leu-Ala-Hea-Ala-Phe-O-PEG₄₀₀₀-OH of Example 11 was removed by a treatment with 4M-hydrochloric acid/dioxane and the obtained product was reacted with
Boc-Leu-Leu-Ala-Hea-ONSu by the same operation under the same conditions as above,
Boc- (Leu-Leu-Ala-Hea)₂-Ala-Phe-O-PEG₄₀₀₀-OH was obtained at a yield of 89 %.

### Example 13

### Manufacture of Boc-Leu-Leu-Ala-Lac- (Leu-Leu-Ala-Hea)₂-Ala-Phe-O-PEG₄₀₀₀-O H

When the Boc protective group of Boc-(Leu-Leu-Ala-Hea)₂-Ala-Phe-O-PEG₄₀₀₀-OH of Example 12 was removed by a treatment with 4M-hydrochloric acid/dioxane and the obtained product was reacted with
Boc-Leu-Leu-Ala-Lac-ONSu by the same operation under the same conditions as above,
Boc-Leu-Leu-Ala-Lac- (Leu-Leu-Ala-Hea)₂-Ala-Phe-O-PEG₄₀₀₀-O H was obtained at a yield of 90 %.

### Example 14

### Manufacture of Boc-(Leu-Leu-Ala-Lac)₂-(Leu-Leu-Ala-Hea)₂-Ala-Phe-O-PEG₄₀₀₀ -OH

When the Boc protective group of
Boc-Leu-Leu-Ala-Lac- (Leu-Leu-Ala-Hea)₂-Ala-Phe-O-PEG₄₀₀₀-O H of Example 13 was removed by a treatment with 4M-hydrochloric acid/dioxane and the obtained product was reacted with Boc-Leu-Leu-Ala-Lac-ONSu by the same operation under the same conditions as above,
Boc- (Leu-Leu-Ala-Lac)₂-(Leu-Leu-Ala-Hea)₂-Ala-Phe-0-PEG₄₀₀₀ -OH was obtained at a yield of 92 %.

### Example 15

### Manufacture of Boc-(Leu-Leu-Ala-Lac)₃-(Leu-Leu-Ala-Hea)₂-Ala-Phe-O-PEG_{400 o}-OH

When the Boc protective group of Boc-(Leu-Leu-Ala-Lac)₂-(Leu-Leu-Ala-Hea)₂-Ala-Phe-0-PEG₄₀₀₀ -OH of Example 14 was removed by a treatment with 4M-hydrochloric acid/dioxane and the obtained product was reacted with Boc-Leu-Leu-Ala-Lac-ONSu by the same operation under the same conditions as above,
Boc- (Leu-Leu-Ala-Lac) ₃- (Leu-Leu-Ala-Hea) ₂-Ala-Phe-0-PEG₄₀₀₀ -OH was obtained at a yield of 91 %. The specific optical rotation of this product was [α] _{D}²⁵ = -11.2 (c = 1.0, CH₃CN) and the NMR spectrum [0.84 ppm (60H) multiple peaks based on two CH₃' s of Leu; 1.1 to 1.7 ppm (66H) multiple peaks based on β-CH₃ of Ala and Lac, β-CH₂ of Leu, γCH and 3 CH₃' s of Boc; 3.5 ppm (360H) single peak based on CH₂ of PEG; 4.2 to 4.9 ppm (24H) multiple peaks based on α-CH of amino acid, lactic acid and glycolic acid; 7.2 ppm (5H) multiple peaks based on aromatic ring hydrogen of Phe; 7.6 to 8.5 ppm (17H) multiple peaks based on NH of amide bond] showed that this was Boc- (Leu-Leu-Ala-Lac)₃-(Leu-Leu-Ala-Hea)₂-Ala-Phe-O-PEG_{400 0}-OH.

### Example 16

### Manufacture of Boc-{Glu(OEt)-Ala-Ala-Lac}₄₋Ala-Phe-OPEG₄₀₀₀-OH

43 g (10 mmol) of HCl·H-Ala-Phe-OPEG₄₀₀₀-OH was dissolved in THF, and 1.1 ml of NMM and 8.6 g (15 mmol) of Boc-Glu(OEt)-Ala-Ala-Lac-ONSu were added and stirred at room temperature for one night. They were treated in accordance with a commonly used method to obtain 41.7 g (89 %) of Boc -Glu (OEt) -Ala-Ala-Lac -Ala- Phe -OPEG₄₀₀₀-OH. The reaction of this Boc-tetradepsipeptide active ester was repeated 3 times to extend the length of the depsipeptide chain so as to obtain
Boc-{Glu(OEt)-Ala-Ala-Lac}₄-Ala-Phe-O-PEG₄₀₀₀-OH in the end.

### Example 17

### Manufacture of Boc-Phe-Lac-(Phe-Leu-Phe-Lac)₂ -Phe-O-PEG₄₀₀₀-OH

38.9 g (0.01 mol) of polyethylene glycol L-phenylalaninate hydrochloride was dissolved in 300 ml of a mixed solvent of acetonitrile (ACN) and tetrahydrofuran (THF) in a weight ratio of 1:1, 1.15 ml (0.0105 mol) of N-methylmorpholine was added to the resulting solution, and 10.4 g (0.015 mol) of t-butoxycarbonyl-L-phenylalanine-L-leucyl L-phenylalanyl-L-lactate N-hydroxysuccinimide ester (Boc-Phe-Leu-Phe-Lac-ONSu) was added to the resulting solution and stirred at room temperature for one night. After the end of a reaction, the solution was concentrated under reduced pressure, the residue was dissolved in dichloromethane, and the solution was rinsed with a 10 % aqueous solution of sodium hydrogen carbonate, a saturated aqueous solution of citric acid and water, and dried on anhydrous sodium sulfate. The solution was concentrated under reduced pressure, and ether was added to the residue to crystallize it.

The obtained Boc-Phe-Leu-Phe-Lac-Phe-O-PEG₄₀₀₀-OH was dissolved in THF, and ether was added to recrystallize it. The yield was 45.4 g (96 %). 47.3 g (10 mmol) of Boc-Phe-Leu-Phe-Lac-Phe-O-PEG₄₀₀₀-OH was treated with 4M-hydrochloric acid/dioxane to remove the Boc protective group. The obtained hydrochloride was dissolved in ACN-THF (1:1), and 1.15 ml of NMM was added. 10.4 g of Boc-Phe-Leu-Phe-Lac-ONSu which is an N-protected tetradepsipeptide active ester was added to the resulting solution and stirred at room temperature for one night. The solution was concentrated under reduced pressure, and the residue was dissolved in dichloromethane, rinsed with a 10 % aqueous solution of sodium hydrogen carbonate, a saturated aqueous solution of citric acid and water, and dried on anhydrous sodium sulfate. The solution was concentrated under reduced pressure, and ether was added to the residue to crystallize it. The obtained crystal was recrystallized with THF-ether to obtain 42.1 (90 %) of pure Boc- (Phe-Leu-Phe-Lac)₂-Phe-O-PEG₄₀₀₀-OH.

This depsipeptide-PEG compound was treated with 4M-HCl/dioxane to remove the Boc group, and the obtained hydrochloride was reacted with Boc-Phe-Lac-ONSu in accordance with a commonly used method to obtain 38.7 g (95 %) of Boc-Phe-Lac-(Phe-Leu-Phe-Lac)₂-Phe-O-PEG₄₀₀₀-OH. The specific optical rotation of this product was [α]_{D}²⁵ = - 12.2 (c=1.0, CH₃CN), and the NMR spectrum [0.84 ppm (12H) multiple peaks based on two CH₃' s of Leu; 1.2 to 1.7 ppm (24H) multiple peaks based on β-CH₃ of Lac, β-CH₂ of Leu, γCH and 3 CH₃'s of Boc; 3.5 ppm (360H) single peak based on CH₂ of PEG; 4.2 to 4.9 ppm (11H) multiple peaks based on α-CH of amino acid and lactic acid; 7.2 ppm (30H) multiple peaks based on aromatic ring hydrogen of Phe; 7. 6 to 8. 5 ppm (8H) multiple peaks based on NH of amide bond] showed that this was
Boc-Phe-Lac-(Phe-Leu-Phe-Lac)₂ -Phe-O-PEG₄₀₀₀-OH.

### Example 18

### Manufacture of micelle

300 mg of Boc- (Leu-Leu-Ala-Lac)₅-Leu-Phe-O-PEG₄₀₀₀-OH and 15 mg of dexamethasone palmitate (DMP) were put into a 100 ml eggplant-shaped flask to be dissolved in 20 ml of acetonitrile. The solvent was distilled off from the solution at 50°C under reduced pressure. 100 ml of water was added to the obtained film-like residue, and the flask was shaken with hands to dissolve the residue in water. Although DMP was insoluble in water, the film-like residue was completely dissolved in water, insoluble matter did not separate out at all, and it was indicated that it was introduced into a micelle. This showed that the block copolymer could solubilize a hardly soluble hydrophobic compound.

### Example 19

### Manufacture of micelle and particle diameter of micelle

200 mg of Boc-(Leu-Leu-Ala-Lac)₅-Leu-Phe-OPEG₄₀₀₀-OH and 60 mg of DMP were dissolved in acetonitrile and treated in accordance with a commonly used method, and 100 ml of physiological saline was added. Since the solution became slightly clouded, it was filtered with a membrane filter. 14 mg of insoluble matter remained on the filter. Therefore, a micelle containing 46 mg of DMP in 100 ml of a physiological saline aqueous solution could be prepared. The diameter of this micelle was 102.5 nm.

### Example 20

### Manufacture of micelle

300 mg g of Boc- (Leu-Leu-Ala-Lac)₃- (Leu-Leu-Ala-Hea)₂-Ala-Phe-OPEG₄₀₀₀ -OH and 10.5 mg of acyclovir (AV) were dissolved in acetonitrile. The solution was concentrated under reduced pressure at 50°C, and 3 ml of water was added to the residue. The residue formed a micelle and easily dissolved.

### Example 21

### Manufacture of micelle

300 mg of Boc- (Leu-Leu-Ala-Lac)₅-Leu-Phe-OPEG₄₀₀₀-OH and 10. 5 mg of acyclovir were dissolved in acetonitrile. The solution was concentrated under reduced pressure at 50°C, and 3 ml of water was added to the residue. The residue formed a micelle and easily dissolved.

### Example 22

### evidence of formation of micelle

A micelle composed of the same amount as in Example 18 of Boc-Phe-Lac-(Phe-Leu-Phe-Lac)₂-Phe-O-PEG₄₀₀₀-OH and the same amount as in Example 18 of DMP was prepared in the same manner as in Example 18 and freeze dried. The freeze dried sample was measured in D₂O and dimethylsulfoxide-d₆ (DMSO-d₆) by 500 MHz proton NMR. In the spectrum obtained by measuring in the D₂O solvent, only a peak derived from the methylene proton of polyethylene oxide and a proton peak derived from water existent in the system were seen at 3.8 ppm and 5.0 ppm, respectively. In the spectrum of the same sample measured in DMSO-d₆, all the proton peaks derived from Boc-Phe-Lac- (Phe-Leu-Phe-Lac)₂-Phe-O-PEG₄₀₀₀-OH and DMP were seen. This fact is considered as follows. When the freeze dried sample was re-dissolved in D₂O, it dissolved while keeping a micelle, only the methylene proton of polyethylene oxide existent external to the micelle was observed and a depsipeptide segment and DMP which were existent internal to the micelle were not observed in the NMR spectrum measured by using a radio wave having weak energy whereas when the same freeze dried sample was dissolved in DMSO-d₆, the micelle was destroyed,
Boc-Phe-Lac- (Phe-Leu-Phe-Lac) ₂-Phe-O-PEG₄₀₀₀-OH and DMP formed a DMSO solution in molecular states, and all the protons were observed by NMR. Therefore, it can be said that the NMR spectrum showed the formation of a micelle composed of Boc-Phe-Lac-(Phe-Leu-Phe-Lac)₂-Phe-0-PEG₄₀₀₀-OH and DMP.

### Example 23

### Evidence of no agglomeration and no destruction of micelle by freeze drying

500 mg of Boc- (Leu-Leu-Ara-Lac)₃(Leu-Leu-Ara-Hea)₂-Ara-Phe-OPEG₄₀₀₀-OH and 100 mg of DMP were dissolved in acetonitrile, and the solvent was distilled off under reduced pressure. 50 ml of water was added to the residue and shaken well. The resulting solution was left to stand for one night, and the formed insoluble matter was filtered with a 450 nm membrane filter. Te weight of the insoluble matter was 24 mg. The filtrate was divided into two, 250 mg (sample 1) of PEG₄₀₀₀ was added to one of them, 125 mg (sample 2) of PEG₄₀₀₀ was added to the other, and both of the samples were freeze-dried. The yields of the samples were 477 mg and 413 mg. When 26.6 mg of the sample 1 was collected and 1 ml of physiological saline was added to the sample 1 and lightly shaken with hands, a transparent homogeneous solution was obtained in 15 seconds. On the other hand, when 20.0 mg of the sample 2 was collected and the same treatment was carried out, after the resulting solution was lightly shaken, it became a homogeneous solution in 45 seconds. These freeze dried products dissolved in physiological saline very quickly to provide transparent homogeneous solutions. The particle diameters of the samples were 105.5 and 101.6 nm.

### Example 24

### Releasability of drug encapsulated in micelle

A micelle solution composed of 100 mg of Boc- (Leu-Leu-Ala-Lac)₃-(Leu-Leu-Ala-Hea)₂-Ala-Phe-OPEG₄₀₀₀ -OH and 5 mg of DMP was prepared and dialyzed with a dialysis cellulose tube having a molecular weight cut off of about 14,000 and an opening diameter of about 5 nm to measure the released DMP by an ultraviolet spectrophotometer. 14 % of DMP was released after 24 hours and 33 % of DMP was released after 48 hours.

### Example 25

### Releasability of drug encapsulated in micelle

A micelle solution composed of 100 mg of Boc- (Leu-Leu-Ala-Lac) ₅-Leu-Phe-O-PEG₄₀₀₀-OH and 5 mg of DMP was prepared and dialyzed with a dialysis cellulose tube having a molecular weight cut off of about 14,000 and an opening diameter of about 5 nm to measure the released DMP by an ultraviolet spectrophotometer. 2 % of DMP was released after 24 hours and 4 % of DMP was released after 48 hours.

### Example 26

### Releasability of drug encapsulated in micelle

A micelle solution composed of 100 mg of Boc- (Leu-Leu-Ala-Lac)₅-Leu-Phe-O-PEG₄₀₀₀-OH and 10 mg of DMP was prepared and dialyzed with a dialysis cellulose tube having a molecular weight cut off of about 14,000 and an opening diameter of about 5 nm to measure the released DMP by an ultraviolet spectrophotometer. 24 % of DMP was released after 24 hours.

### Example 27

### Releasability of drug encapsulated in micelle

A micelle solution composed of 100 mg of Boc- (Leu-Leu-Ala-Lac) ₅-Leu-Phe-O-PEG₄₀₀₀-OH and 5 mg of dexamethasone was prepared and dialyzed with a dialysis cellulose tube having a molecular weight cut off of about 14, 000 and an opening diameter of about 5 nm to measure the released DMP by an ultraviolet spectrophotometer. 57 % of DMP was released after 10 hours and only 3 % of DMP was released 10 hours after that. It is understood from these that compatibility between the drug and the block copolymer of the present invention greatly differs for each drug and that the formation of a micelle can be controlled by constructing the polymer structure of the hydrophobic segment in the micelle for each drug so that it becomes compatible with the chemical properties of the drug. It is also understood that the releasability of the drug encapsulated in the micelle can be controlled by the structure of the block copolymer of the present invention and the amount of the drug to be encapsulated.

### Example 28

### Synthesis of condensate of Boc-(Leu-Leu-Ala-Lac)₅-Leu-Phe-0-PEG₄₀₀₀-OH and folic acid

4.4 g (0.01 mol) of folic acid was dissolved in 100 ml of acetic acid, 1.2 g (0.01 mol) of thionyl chloride was added dropwise to the resulting solution at 0°C, and the obtained solution was added dropwise to 300 ml of a THF solution containing (0.005 mol) of Boc-(Leu-Leu-Ala-Lac)₅-Leu-Phe-O-PEG₄₀₀₀-OH and 4 ml of pyridine and reacted at room temperature for 3 hours. The obtained solution was concentrated under reduced pressure, dissolved in ethyl acetate, rinsed with water and an aqueous solution of sodium hydrogen carbonate and dried on anhydrous sodium sulfate. The obtained solution was concentrated under reduced pressure, and ether was added to the solution to precipitate the formed product. The product was re-precipitated from THF to obtain a purified condensate of Boc-(Leu-Leu-Ala-Lac)₅-Leu-Phe-O-PEG₄₀₀₀-OH and folic acid.

### Example 29

### Manufacture of particulate

200 mg of Boc-Leu-Leu-Ala-Lac-Leu-Leu-Lys(Z)-Lac-Leu-Lac-Phe-O-PEG ₆₀₀-O-Phe-Lac-Leu-Lac-Lys(Z)-Leu-Leu-Lac-Ala-Leu-Leu-Boc and 10 mg of dexamethasone palmitate were dissolved in 2 ml of acetonitrile-THF (1:1) and stirred with a stirrer at a high speed, and water was added gradually to prepare 100 ml of a solution. The obtained solution was stirred for 30 minutes. The solution became clouded, and a particulate containing the drug was prepared.

### Example 30

### Manufacture of composite

10 mg of dexamethasone palmitate was put into a mortar, 1 ml of an aqueous solution obtained by dissolving 100 mg of Boc-(Leu-Leu-Ala-Lac)₅-Ala-Phe-O-PEG₄₀₀₀-OH in 15 ml of water and subjecting the resulting solution to an ultrasonic treatment was added to and kneaded with the dexamethasone palmitate by a pestle for 1 minute, whereby dexamethasone palmitate crystals were uniformly dispersed in a micro-crystal state, and the obtained suspension was fully stable at room temperature. When 10 mg of dexamethasone palmitate was put into an agate mortar and 1 ml of water was added to and kneaded with the dexamethasone palmitate by an agate pestle, dexamethasone palmitate had no affinity for water, remained as a big mass and did not disperse. It is understood from this that the block copolymer of the present invention can form a stable composite with drug micro-crystals.

### Example 31

### Sustention of pharmacological effect

An injection solution (physiological saline) containing a micelle composed of Boc- (Leu-Leu-Ala-Lac)₅-Leu-Phe-O-PEG₄₀₀₀-OH obtained in the same manner as in Example 18 and dexamethasone palmitate was prepared and injected into male SD rats (5 weeks old, n = 3 to 5) intravenously to ensure that the dose of dexamethasone became 0.5 mg/kg. 7 days after the administration of the drug, 0.1 ml of a 3 % carrageenan solution was injected into the right feet of the rats subcutaneously to measure an increase in the volume of each foot by edema after 4 hours. As comparison, the same doses as above of a lipid micro-sphere injection solution of dexamethasone palmitate (Rimetasone (registered trademark) of Mitsubishi Pharma Corporation), a sodium dexamethasone phosphate injection solution (Decadron (registered trademark) of Banyu Pharmaceutical Co., Ltd.) and physiological saline containing no drug were administered in place of the above micelle to measure the volume of each foot on the same time schedule. The volume of the foot increased by 19.9±7.7 % in case of physiological saline containing no drug and 15.4±3.5 % in case of Rimetasone. However, it increased by 1.46±1.46 % in the case of the above micelle and a significant (P<0.01) anti-inflammatory effect was observed as compared with physiological saline and Rimetasone. In the experiment using the sodium dexamethasone phosphate injection solution (Decadron (registered trademark) of Banyu Pharmaceutical Co., Ltd.), the strongest anti-inflammatory effect was observed after 24 hours and an increase in the volume of the foot was significantly suppressed but its effect was lost on the 3^{rd} day or later. The sustention of the pharmacological effect of the micelle of the present invention became evident.

### Example 32

### Manufacture of micelle

6 mg of Boc- (Leu-Leu-Ala-Lac) ₅-Leu-Phe-O-PEG₄₀₀₀-OH and 0.12 mg of prostaglandin I₂ (PGI₂) were put into a 100 ml eggplant-shaped flask and dissolved in 30 ml of acetonitrile-THF-ethanol (5:5:1). The solvent was distilled off from this solution under reduced pressure. 30 ml of distilled water was added to the obtained film to dissolve it in water. The film dissolved in water completely. Then the UV spectrum of this solution was measured. Even after 3 days, the UV spectrum having an absorption maximum at 195 nm did not change. 3 mg of PEG4000 was added to this aqueous solution and freeze dried. Even after the freeze dried product was kept at room temperature for 24 hours, its UV spectrum did not change.

### Example 33

### Manufacture of micelle

50 mg of Boc- (Leu-Leu-Ala-Lac)₅-Leu-Phe-O-PEG₄₀₀₀-OH and 5.0 mg of beraprost sodium which is prostacyclin were put into a 100 ml eggplant-shaped flask and dissolved in 30 ml of acetonitrile-THF-ethanol (5:5:1). The solvent was distilled off from this solution under reduced pressure. 1 ml of an acetic acid buffer (0.1 M) having a pH of 4.5 was added to the obtained solid to dissolve it by applying ultrasonic waves. Although a precipitate was produced at this pH with beraprost sodium alone, the solid dissolved in the buffer at this pH completely, which indicated that it was introduced into a micelle.

### Example 34

### Manufacture of micelle

2.2 mg of paclitaxel and 55 mg of Boc- (Leu-Leu-Ala-Lac)₅-Leu-Phe-O-PEG₄₀₀₀-OH were dissolved in 10 ml of acetonitrile, and the solvent was distilled off under reduced pressure.

A transparent film remaining on a glass wall was dissolved in 30 ml of water at 40°C. Although paclitaxel is insoluble in water, the film dissolved in water completely and insoluble matter did not separate out at all, which indicated that it was introduced into a micelle. The transparent homogeneous solution was filtered with a membrane filter, and 25 mg of PEG4000 was added to the filtrate to freeze-dry it. The freeze-dried sample was measured in D₂O and dimethyl sulfoxide-d₆ (DMSO-d₆) by 50 MHz proton NMR. In the spectrum obtained by measuring in the D₂O solvent, only a peak derived from the methylene proton of polyethylene oxide and a proton peak derived from water existent in the system were seen at 3.8 ppm and 5.0 ppm, respectively. In the spectrum of the same sample in DMSO-d₆, all proton peaks derived from Boc- (Leu-Leu-Ala-Lac) ₅-Leu-Phe-O-PEG₄₀₀₀-OH and paclitaxel were seen. This fact is considered as follows. When the freeze dried sample was re-dissolved in D₂O, it dissolved while keeping a micelle, only the methylene proton of polyethylene oxide existent external to the micelle was observed and a depsipeptide segment and paclitaxel which were existent internal to the micelle were not observed in the NMR spectrum measured by using a radio wave having weak energy whereas when the same freeze dried sample was dissolved in DMSO-d₆, the micelle was destroyed,
Boc- (Leu-Leu-Ala-Lac) ₅-Leu-Phe-O-PEG₄₀₀₀-OH and paclitaxel formed a DMSO solution in molecular states, and all the protons were observed by NMR. Therefore, it can be said that the NMR spectrum showed that a micelle composed of Boc- (Leu-Leu-Ala-Lac) ₅-Leu-Phe-O-PEG₄₀₀₀-OH and pictaxel was formed.

### Example 35

### Manufacture of micelle

20 mg of methotrexate and 200 mg of Boc-(Leu-Leu-Ala-Lac)₅-Leu-Phe-O-PEG₄₀₀₀-OH were dissolved in 30 ml of THF-methanol (1:1) , and the solvent was distilled off under reduced pressure. 50 ml of water was added to the remaining transparent film to dissolve it. Although methotrexate is almost insoluble in water, the film dissolved in water completely and insoluble matter did not separate out at all, which indicated that it was introduced into a micelle. The transparent homogeneous solution was filtered with a membrane filter, and 100 mg of PEG4000 was added to the filtrate to freeze-dry it. The freeze-dried sample was measured in D₂O and dimethyl sulfoxide-d₆ (DMSO-d₆) by 500 MHz proton NMR. In the spectrum obtained by measuring in the D₂O solvent, only a peak derived from the methylene proton of polyethylene oxide and a proton peak derived from water existent in the system were seen at 3.8 ppm and 5.0 ppm, respectively. In the spectrum of the same sample measured in DMSO-d₆, all proton peaks derived from
Boc-(Leu-Leu-Ala-Lac)₅-Leu-Phe-0-PEG₄₀₀₀-OH and methotrexate were seen. Therefore, it can be said that this NMR spectrum showed that a micelle composed of
Boc- (Leu-Leu-Ala-Lac)₅-Leu-Phe-O-PEG₄₀₀₀-OH and methotrexate was formed.

### Example 36

### Synthesis of Boc-(Leu-Leu-Ala-Lac)₅-Leu-D-Ala-0- PEG₄₀₀₀-OH

20 g (5 mmol) of HO-PEG₄₀₀₀-OH was dissolved in 300 ml of tetrahydrofuran (THF), and 4.3 g (15 mmol) of t-butoxycarbonyl-D-alanine-D-hydroxysuccinate (Boc-D-Ala-ONSu) and 0.19 g (1.5 mmol) of 4-dimethylaminopyridine were added to the resulting solution and stirred at room temperature for 3 days. The obtained solution was concentrated under reduced pressure, the obtained oil was dissolved in 400 ml of dichloromethane, and the resulting solution was rinsed with a 10 % aqueous solution of citric acid, a saturated aqueous solution of sodium hydrogen carbonate and saturated saline and dried on anhydrous sodium sulfate. When the solution was concentrated under reduced pressure and ether was added to the obtained oil, it crystallized. The crystal was dissolved in hot THF, and ether was added to recrystallize it. The yield was 20 g (92 %). When 20 g (4.6 mmol) of Boc-D-Ala-O-PEG₄₀₀₀-O-D-Ala-Boc was treated with 4M-HCl in dioxane and the solvent was distilled off under reduced pressure, the solid residue was obtained. Ether was added to the residue, and the obtained crystal was filtered. The NMR spectrum showed that this crystal was HCl. H-D-Ala-O-PEG-OH. The yield was 17.6 g (93%). The obtained crystal was treated in the same manner as in Reference Example 2 and Examples 1 to 4 to synthesize Boc- (Leu-Leu-Ala-Lac)₅-Leu-D-Ala-O-PEG₄₀₀₀-OH.

### Example 37

### Synthesis of Boc-(Leu-Leu-Ala-Lac)₅-Leu-Lys(Z)-O-PEG₄₀₀₀-OH

20 g (5 mmol) of HO-PEG₄₀₀₀-OH was dissolved in 300 ml of tetrahydrofuran (THF), and 7.2 g (15 mmol) of t-butoxycarbonyl-benzyloxycarbonyl-L-lysine N-hydroxysuccinate (Boc-Lys(Z)-ONSu) and 0.19 g (1.5 mmol) of 4-dimethylaminopyridine were added to the resulting solution and stirred at room temperature for 3 days. The obtained solution was concentrated under reduced pressure, the obtained oil was dissolved in 400 ml of dichloromethane, and the resulting solution was rinsed with a 10 % aqueous solution of citric acid, a saturated aqueous solution of sodium hydrogen carbonate and saturated saline and dried on anhydrous sodium sulfate. When the solution was concentrated under reduced pressure and ether was added to the obtained oil, it crystallized. The crystal was dissolved in hot THF, and ether was added to recrystallize it. The yield was 21 g (89 %). When 18.9 g (4 mmol) of Boc-Lys (Z) -O-PEG₄₀₀₀-O-Lys (Z)-Boc was treated with 4M-HC1 in dioxane and the solvent was distilled off under reduced pressure, the solid residue was obtained. Ether was added to the residue, and the obtained crystal was filtered. The NMR spectrum showed that this crystal was HCl· H-Lys(Z)-O-PEG-OH. The yield was 16.5 g (96 %). The obtained crystal was treated in the same manner as in Reference Example 2 and Examples 1 to 4 to synthesize Boc-(Leu-Leu-Ala-Lac)₅-Leu-Lys(Z)-O-PEG₄₀₀₀-OH.

### Example 38

### Synthesis of Boc- (Leu-Leu-Ala-Lac)₅-Leu-Asp (OEt) -O-PEG₄₀₀₀-OH

20 g (5 mmol) of HO-PEG₄₀₀₀-OH was dissolved in 300 ml of tetrahydrofuran (THF), and 5.4 g (15 mmol) of t-butoxycarbonyl-ethyl-L-aspartate N-hydroxysuccinate (Boc-Asp(OEt)-ONSu) and 0.19 g (1.5 mmol) of 4-dimethylaminopyridine were added to the resulting solution and stirred at room temperature for 3 days. The obtained solution was concentrated under reduced pressure, the obtained oil was dissolved in 400 ml of dichloromethane, and the resulting solution was rinsed with a 10 % aqueous solution of citric acid, a saturated aqueous solution of sodium hydrogen carbonate and saturated saline and dried on anhydrous sodium sulfate. When the solution was concentrated under reduced pressure and ether was added to the obtained oil, it crystallized. The crystal was dissolved in hot THF, and ether was added to recrystallize it. The yield was 19 g (85 %). When 18 g (4.01 mmol) of Boc-Asp (OEt) -O-PEG₄₀₀₀-O-Asp (OEt) -Boc was treated with 4M-HCl in dioxane and the solvent was distilled off under reduced pressure, the solid residue was obtained. Ether was added to the residue, and the obtained crystal was filtered. The NMR spectrum showed that this crystal was HC1-H-Asp(OEt)-O-PEG-OH. The yield was 15.1 g (90 %). The obtained crystal was treated in the same manner as in Reference Example 2 and Examples 1 to 4 to synthesize Boc- (Leu-Leu-Ala-Lac)₅-Leu-Asp(OEt) -O-PEG₄₀₀₀-OH.

### Example 39

### Test on toxicity by single dose intravenous administration of Boc- (Leu-Leu-Ala-Lac)₅-Leu-Phe-O-PEG₄₀₀₀-OH into guinea pigs

Hartley male guinea pigs which were 10 weeks old and weighed 338 to 490 g were used. Boc- (Leu-Leu-Ala-Lac)₅-Leu-Phe-O-PEG₄₀₀₀-OH (applied dose of 20 mg/kg) as a test substance, HO-PEG4000-OH (applied dose of 10 mg/kg) as a control and physiological saline as a negative control were used. These substances were dissolved in physiological saline to a predetermined concentration, subjected to an ultrasonic treatment and sterilized by filtration with a 0.22 µm membrane filter. The physiological saline was also sterilized by filtration with a 0.22 µm membrane filter. Physiological saline, HO-PEG₄₀₀₀-OH and Boc- (Leu-Leu-Ala-Lac)₅-Leu-Phe-O-PEG₄₀₀₀-OH were administered into the veins of the front feet of a group of 5 guinea pigs at a dose of 1 ml/kg over 1 minute and observed for 24 hours. 1 month after that, the weights of the guinea pigs were measured and the general conditions of the guinea pigs were observed once a week. As a result, no guinea pigs died. Any special abnormality in the general conditions was not seen in the guinea pigs. No appreciable changes in the weights were seen. It was understood from these that toxicity by the single dose intravenous administration of Boc-(Leu-Leu-Ala-Lac)₅-Leu-Phe-O-PEG₄₀₀₀-OH to the guinea pigs was not observed.

### Example 40

### Test on active systemic anaphylaxis (ASA) reaction using guinea pigs administered with

### Boc- (Leu-Leu-Ala-Lac)₅-Leu-Phe-O-PEG₄₀₀₀-OH

Hartley guinea pigs which were 7 weeks old and weighed 380 to 510 g were used. For animal sensitization, Boc- (Leu-Leu-Ala-Lac) ₅-Leu-Phe-O-PEG₄₀₀₀-OH (applied dose of 10 mg/kg) as a test substance, egg albumin (applied dose of 10 mg/kg) as a positive control and physiological saline as a negative control were used. These substances were dissolved in physiological saline to a predetermined concentration, and the same amount of FCA (Freund' s complete adjuvant) or FIA (Freund's incomplete adjuvant) was added to prepare a w/o emulsion. The FCA emulsion was first administered and then the FIA emulsion was administered under the skin of the back and foot pad of each guinea pig at a dose of 2.5 ml/kg on the second and third weeks for sensitization. For an induction reaction, Boc- (Leu-Leu-Ala-Lac) ₅-Leu-Phe-O-PEG₄₀₀₀-OH (applied dose of 4.0 mg/kg) as a test substance, egg albumin (applied dose of 0.4 mg/kg) as a positive control and physiological saline as a negative control were used. These substances were dissolved in physiological saline to a predetermined concentration. The resulting solution was administered into the veins of the front feet or rear feet of the guinea pigs at a dose of 1.0 ml/kg on the fifth week for induction and observed for 4 hours. For 1 month after that, the weights of the guinea pigs were measured and the general conditions of the guinea pigs were observed once a week. As for the number of animals, 4 guinea pigs were administered with Boc-(Leu-Leu-Ala-Lac)₅-Leu-Phe-0-PEG₄₀₀₀-OH, 3 guinea pigs were administered with egg albumin and 3 guinea pigs were administered with physiological saline. As a result, the guinea pigs administered with Boc-(Leu-Leu-Ala-Lac)₅-Leu-Phe-O-PEG₄₀₀₀-OH did not show a anaphylaxis reaction. Any special abnormality was not seen in the general conditions and weights of the guinea pigs. An anaphylaxis symptom was seen in all the guinea pigs as the positive control right after induction administration. It is understood from these that the guinea pigs administered with Boc-(Leu-Leu-Ala-Lac)₅-Leu-Phe-O-PEG₄₀₀₀-OH were negative in the active systemic anaphylaxis (ASA) reaction test and had no antigenicity.

## Claims

1. A biocompatible block copolymer having a hydrophobic segment composed of amino acid and hydroxycarboxylic acid and a hydrophilic segment composed of polyalkylene oxide, wherein the terminal not bonded to the hydrophilic segment of the hydrophobic segment is composed of an amino acid unit.

2. The biocompatible block copolymer according to claim 1 which is represented by the following formula (1): 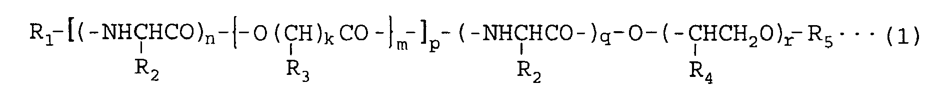 wherein R₁ is a hydrogen atom, alkyl group, substituted alkyl group or protective group for amino group, R₂ is the side chain of natural amino acid or derivative group thereof, R₃ is a hydrogen atom, alkyl group or substituted alkyl group, R₄ is a hydrogen atom or methyl group, R₅ is a hydrogen atom, alkyl group, substituted alkyl group, ligand residue, ligand residue having a coupling group, or group represented by 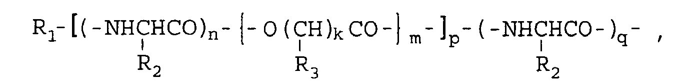 n is an integer of 1 to 20, k is an integer of 1 to 6, m is an integer of 1 to 20, p is an integer of 1 to 20, q is an integer of 0 to 20, and r is an integer of 2 to 870.

3. The biocompatible block copolymer according to claim 1 or 2, wherein the amino acid is at least one amino acid selected from the group consisting of alanine, valine, leucine, isoleucine, phenylalanine, glutamic acid, aspartic acid, lysine, arginine and derivatives in the side chains of these amino acids.

4. The biocompatible block copolymer according to any one of claims 1 to 3, wherein the hydroxycarboxylic acid is at least one hydroxycarboxylic acid selected from the group consisting of glycolic acid, lactic acid, hydroxybutyric acid, hydroxyvaleric acid, hydroxycaproic acid and hydroxycapric acid.

5. The biocompatible block copolymer according to any one of claims 1 to 4, wherein the polyalkylene oxide of the hydrophilic segment is polyethylene oxide.

6. The biocompatible block copolymer according to any one of claims 1 to 5, wherein the number average molecular weight of the polyalkylene oxide of the hydrophilic segment is 10,000 or less.

7. The biocompatible block copolymer according to any one of claims 1 to 6, wherein the polyalkylene oxide of the hydrophilic segment is a poly(ethylene oxide/propylene oxide) block copolymer.

8. The biocompatible block copolymer according to claim 1, wherein the hydrophobic segment is composed of a depsipeptide.

9. The biocompatible block copolymer according to claim 2, wherein the ligand forming the ligand residue represented by R₅ in the formula (1) is lectin, antibody, antibody fragment, hormone, adhesion factor, transferrin or folic acid.

10. A micelle having an inner core comprising the hydrophobic segment of the biocompatible block copolymer of any one of claims 1 to 9 and an outer shell comprising the hydrophilic segment of the biocompatible block copolymer.

11. A micelle comprising the biocompatible block copolymer of any one of claims 1 to 9 and a drug, wherein the inner core of the micelle comprises the hydrophobic segment and the drug and the outer shell comprises the hydrophilic segment of the biocompatible block copolymer.

12. A particulate (excluding a micelle) comprising the biocompatible block copolymer of any one of claims 1 to 9 and a drug.

13. A composite (excluding a micelle) comprising the biocompatible block copolymer of any one of claims 1 to 9 and a drug.

14. The micelle according to claim 10 or 11 which has an average particle diameter of 5 to 300 nm.

15. The micelle, particulate or composite according to any one of claims 11 to 14, wherein the drug is an anti-inflammatory drug, defervescent, analgesic drug, antarthritic/antirheumatic drug, antigout drug, cardiac restorative, antianginal drug, beta-adrenergic blocking drug, calcium antagonist drug, antiarrhythmic drug, diuretic, antihypertensive, drug for improving the disturbances of peripheral circulation, antithrombogenic drug, antihyperlipemic drug, antiplatelet drug, pressor drug, drug for improving the metabolism of cerebral circulation, anticancer drug, immunosuppressive drug, antiallergic drug, antihistamine drug, bronchodilator, antiasthmatic drug, antitussive drug, expectorator, antiulcer drug, antidiarrheal, antiemetic, antidiabetic, hypnotic/sedating drug, antianxiety drug, antipsychotic drug, antidepressant, antivertigo drug, antiepilepsy drug, muscle relaxant drug, antiparkinson drug, autonomic effect drug, antibiotic, antibacterial drug, antifungal drug, antiviral, anthelmintic, hormonal drug, osteoprosis preventing and bone metabolism improving drug, vitamin drug, hemostatic, enzyme drug, vaccine, anesthetic, diagnostic product, contrast drug or test drug.

16. The micelle, particulate or composite according to any one of claims 11 to 14, wherein the drug is a hydrophobic drug.

17. A pharmaceutical composition comprising the micelle, particulate or composite of any one of claims 11 to 14.

18. The pharmaceutical composition according to claim 17 which is administered as an injectable, oral, inhalant, transdermal or transmucosal preparation.

19. A method of manufacturing a micelle or composite comprising a drug, which method comprises the step of mixing a biocompatible block copolymer having a hydrophobic segment composed of amino acid and hydroxycarboxylic acid and a hydrophilic segment composed of polyalkylene oxide with a drug in water.

20. A method of manufacturing a micelle, particulate or composite, which method comprises the steps of providing a mixture of a biocompatible block copolymer having a hydrophobic segment composed of amino acid and hydroxycarboxylic acid and a hydrophilic segment composed of polyalkylene oxide, a drug and a solvent, and removing the solvent from the mixture.

21. Use of the biocompatible block copolymer of claim 1 in combination with a drug in a living body.

22. Use of the biocompatible block copolymer of claim 1 in combination with a drug for the manufacture of a micelle, particulate or composite.
